# EUROPEAN PATENT SPECIFICATION

(11) **EP 4 204 491 B1**
(45) Date of publication and mention of the grant of the patent: **23.09.2026**
(21) Application number: 21860739.8
(22) Date of filing: 18.08.2021
(51) Int. Cl.: C08K 5/3492, C08K 3/32, C08K 3/016, C09K 21/10, C09K 21/12, C09K 21/14, C07D 251/70, C07F 9/38, C07F 9/6593, C08G 18/28, C08G 18/38, C08G 18/73, C08G 18/75, C08G 18/76, C08G 69/26, C08K 5/5399, C08G 77/26

(54) **NON-HALOGENATED MULTI-MELAMINE FLAME-RETARDANT COMPOUNDS AND SALTS THEREOF AND PROCESSES FOR THEIR PREPARATION**
HALOGENFREIE MULTIMELAMIN-FLAMMSCHUTZMITTEL UND SALZE DAVON UND VERFAHREN ZU IHRER HERSTELLUNG
COMPOSÉS IGNIFUGES MULTI-MÉLAMINE NON HALOGÉNÉS ET LEURS SELS ET PROCÉDÉS POUR LEUR PRÉPARATION

(30) Priority: 26.08.2020 IL 27696420
(43) Date of publication of application: 05.07.2023
(73) Proprietor: State of Israel Prime Minister's Office Israel Institute for Biological Research, 7410001 Nes-Ziona (IL)
(72) Inventor: MIZRAHI, Dana, 7576417 Rishon LeZion (IL)
(74) Representative: Kancelaria Eupatent.pl Sp. z o.o.
(86) International application number: PCT/IL2021/051007
(87) International publication number: WO 2022/043990

(56) References cited:
- CN-A- 101 786 637
- CN-A- 102 816 346
- CN-A- 105 330 803
- CN-A- 106 751 380
- CN-A- 106 832 778
- CN-A- 110 982 165
- IL-A- 234 250
- JP-A- 2017 002 402
- JP-A- S5 667 360
- US-A1- 2004 039 085
- US-A1- 2006 247 339
- US-A1- 2011 152 431
- US-A1- 2018 105 618

## Description

### FIELD OF THE INVENTION

The present invention relates to the field of flame-retardant compounds. More particularly, the invention relates to non-halogenated melamine compounds and salts thereof for use as flame-retardants and to processes for their preparation.

### BACKGROUND OF THE INVENTION

There is a constant need for effective and environmentally-friendly flame retardants. To date, many of the currently used flame retardants are beset by major deficiencies in their practical use such as exhibiting undesirable decomposition temperatures, which are characterized by heat stabilities over a very particular and narrow range of temperatures, and difficult application procedures require their presence at high concentrations thereby adversely affecting the desirable properties of the final manufactured product to which the flame retardant is applied. In addition, the preparation processes for many of the available flame retardants are inefficient and, hence, usually expensive.

Melamine and many of its derivatives have long been known for their useful flame-retarding properties owing to their facile release of nitrogen gas when subjected to high temperatures or when ignited producing relatively inert decomposition products. Melamine and its derivatives have therefore been frequently used as main retarding components or auxiliary additives in paints, plastics, paper and fabrics to afford products with good thermal stability and improved flame-resistance. However, the present melamine-based flame retardants suffer from a number of drawbacks, including, inter alia, non-optimal decomposition temperatures, and undesirable sublimation, leeching or otherwise bleeding of the flame-retarding compounds at various temperatures during processing or out of the surface of the final product, which significantly limits their efficiency and utility in many applications and oftentimes degrades the appearance of the final product. Thus, compounds which exhibit better flame retardant properties such as greater thermal stability and improved decomposition characteristics have been sought.

CN101786637 discloses materials and a method for preparing highly flame-retardant organically intercalated layered clays. The method involves inserting melamine or melamine derivatives into layered clay structures, in particular montmorillonite, by cation exchange. The intercalating agents are salts formed from melamine or its derivatives and organic acids. Preparation of the intercalated clay comprises steps including mixing of the starting materials, dispersion, cation exchange and grinding. The resulting organic layered clay is described as being dispersible in polymer matrices and suitable for use as an expandable flame-retardant additive or as a component in the manufacture of flame-retardant plastics, rubbers, films, coatings and foamed materials.

JPS5667360A discloses a polyamide resin composition intended for the production of moulded articles with improved heat resistance, flame retardancy and mechanical strength. The composition comprises a polyamide resin blended with a melamine-based additive obtained by reacting melamine with a diisocyanate. The reaction product has a repeating structure defined by an aliphatic, aromatic or alicyclic hydrocarbon group (C₁-C₂₀) and an integer parameter n representing the degree of repetition. While compounds with n = 1 are readily synthesised and compounds with n in the range of 1 to 5 are considered practically obtainable, the synthesis becomes increasingly difficult as n increases, with an upper practical limitation reported for values of n exceeding approximately 15.

CN110982165A discloses an EVA-based environmentally friendly colour masterbatch comprising a pigment, a pigment carrier, a dispersing agent, talcum powder, a flame-retardant additive, modified bamboo fibres, an auxiliary agent and a light stabiliser in specified weight proportions. The masterbatch is prepared by premixing the components, melt-processing in a screw extruder, followed by cooling and granulation. The composition is described as being compatible with EVA matrices and suitable for providing coloration together with flame-retardant properties in EVA-based materials.

CN106832778A discloses a preparation method for a halogen-free flame-retardant epoxy resin system in which an epoxy resin and an anhydride curing agent are respectively modified using a halogen-free flame-retardant compound containing hydroxyl or amino functional groups. The method is directed to producing epoxy systems suitable for use in encapsulation and insulation applications, including the bonding and packaging of electronic components, casting processes and automatic pressure gelation (APG). The resulting epoxy resin compositions are described as exhibiting flame-retardant behaviour meeting **UL** 94 V-0 classification at thicknesses above approximately 1.5 mm.

CN105330803A discloses a triazine-based hyperbranched polyurea charring agent and a method for its preparation. The charring agent is synthesised by reacting isocyanate groups (-NCO) with amino groups (-NH₂) of melamine in a solvent, thereby forming a hyperbranched polymer structure containing triazine rings and aromatic units. The material is described as exhibiting thermal stability suitable for polymer processing and improved compatibility with polymer matrices. The charring agent is intended for use in flame-retardant polymer compositions, in particular in combination with ammonium polyphosphate as an acid source, for example in polypropylene-based systems.

It is an object of the present invention to provide compounds which are capable of overcoming the shortcomings of existing flame retardants and which can be incorporated into existing resins, polymers and compositions to improve their thermal stability during preparation or as end use of the final product at elevated temperatures.

It is another object of the invention to provide processes for the preparation of the compounds of the invention as well as methods of using the same.

Other objects and advantages of the invention will become apparent as the description proceeds.

### SUMMARY OF THE INVENTION

The present disclosure relates to non-halogenated melamine compounds for use as flame retardants, said compounds being characterized by having a TGA₅₀ value of at least 300 degrees Centigrade, or a TGA₂₅ value of at least 200 degrees Centigrade, or a TGA₇₅ value of at least 500 degrees Centigrade, as measured on a thermogravimetric analysis (TGA) curves produced at a heating rate of 10 degrees Centigrade per minute and at a 60 mL/min oxygen or nitrogen flow, and wherein said compounds are selected from small molecules, linear or branched polymers, or salts thereof. As further described below, it was surprisingly found that compounds having the specific TGA values detailed above exhibit enhanced flame retardant properties.

The invention is defined by the appended claims.

In some aspects, the non-halogenated melamine compounds for use as flame retardants have a structure according to formula (I):

A-(Y-X)ₙ (I)

Wherein,
A is absent or is a 6-membered ring selected from 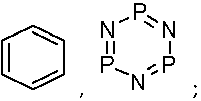 or A is n-butyl or 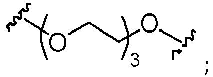
Y is absent or is attached to A via a carbon or phosphorous atom present on A, and is selected from C=O, CO-(CH₂)ₚ-CO, 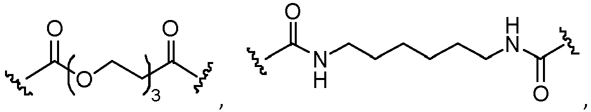 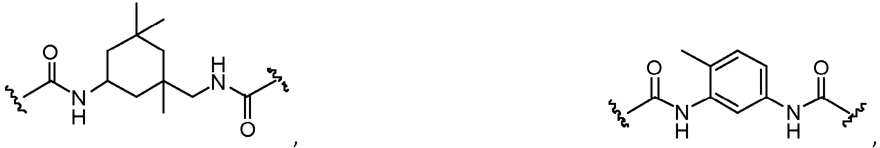 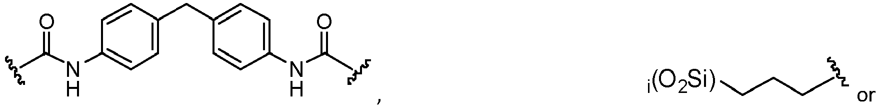 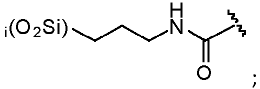
wherein p is an integer from 0-4 and ᵢ(O₂Si) denotes bulk silica;
X is a melamine moiety of the formula: 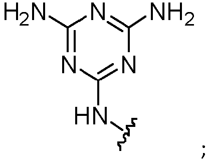 or
X is Xₘ, wherein Xₘ represents a branched repeating unit of a melamine moiety having a structure according to formula (II): 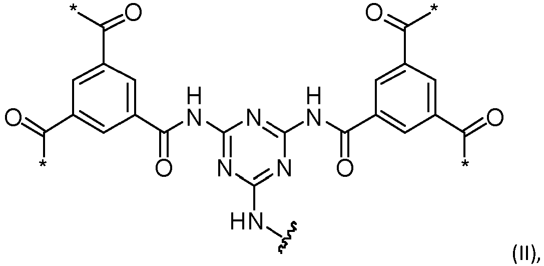
or according to formula (III): 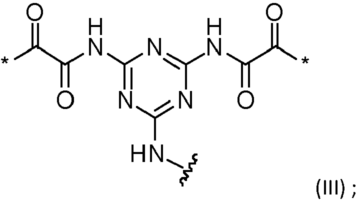
wherein:
   m is an integer from 1-500;
   the asterisk (*) denotes the point of attachment in X to which additional X groups attach as depicted in each of formulas (II) and (III) above;
   X is attached to Y via an amino group present on X if Y is present;
   X is attached to either a carbon or a phosphorous atom present on A via an amino group present on X if Y is absent; and
   n is an integer from 1-500.

According to a further specific embodiment, the invention provides a non-halogenated melamine compound for use as a flame retardant, wherein A is 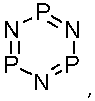 Y is absent, X is a melamine moiety, and n is 6, such that the compound has the structure below (compound A):

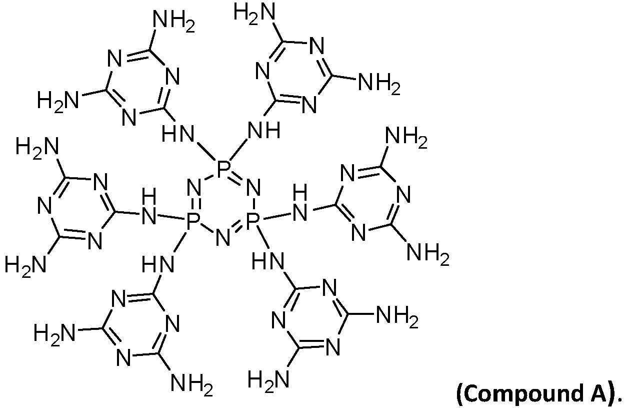

According to yet another specific embodiment, the invention provides a non-halogenated melamine compound for use as a flame retardant with the aforesaid TGA characteristics, wherein A is absent, Y is C=O, and X is a melamine moiety, such that the compound has the structure below (compound B): 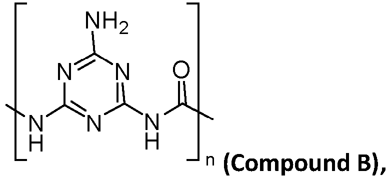 wherein n is an integer from 1-100.

According to a further specific embodiment, the invention provides a non-halogenated melamine compound for use as a flame retardant with the aforesaid TGA characteristics, wherein A is absent, Y is oxalyl, and X is a melamine moiety, such that the compound has the structure below (compound J): 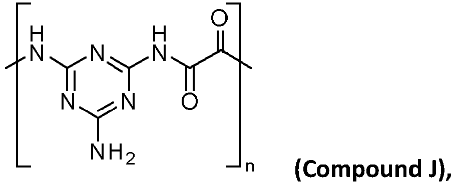 wherein n is an integer from 1-100.

According to a further aspect there is discussed a non-halogenated melamine compound for use as a flame retardant with the aforesaid TGA characteristics, wherein A is absent, Y is 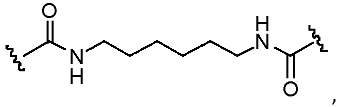 and X is a melamine moiety, such that the compound has the structure below (compound M): 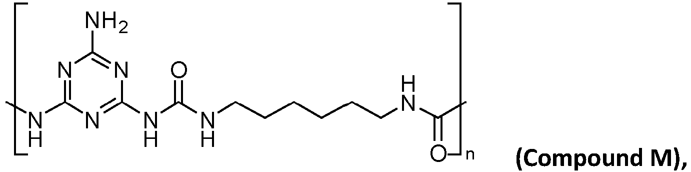 Wherein n is an integer from 1-100.

According to yet another aspect there is discussed a non-halogenated melamine compound for use as a flame retardant with the aforesaid TGA characteristics, wherein A is absent, Y is 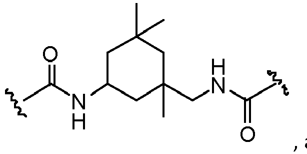 and X is a melamine moiety, such that the compound has the structure below (compound N):

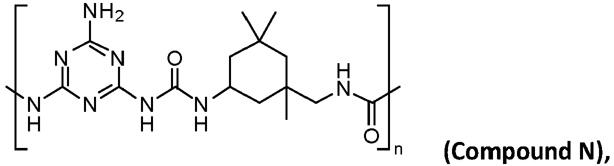

Wherein n is an integer from 1-100.

According to a further aspect there is discussed a non-halogenated melamine compound for use as a flame retardant with the aforesaid TGA characteristics, wherein A is absent, Y is 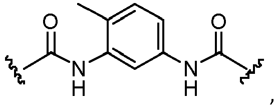 and X is a melamine moiety, such that the compound has the structure below (compound O):

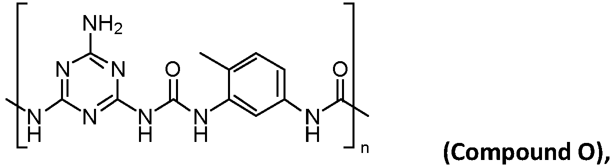

Wherein n is an integer from 1-100.

According to another aspect there is discussed a non-halogenated melamine compound for use as a flame retardant with the aforesaid TGA characteristics, wherein A is absent, Y is 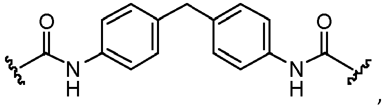 and X is a melamine moiety, such that the compound has the structure below (compound P):

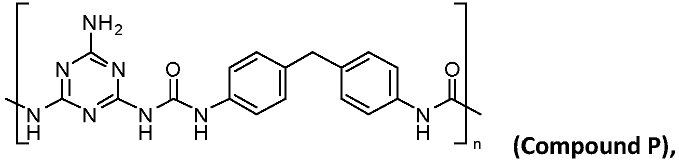

Wherein n is an integer from 1-100.

According to another specific embodiment, the invention provides a non-halogenated melamine compound for use as a flame retardant with the aforesaid TGA characteristics, wherein A is *n-*butyl, Y is C=O, X is a melamine moiety, and n is 2, such that the compound has the structure below (compound C):

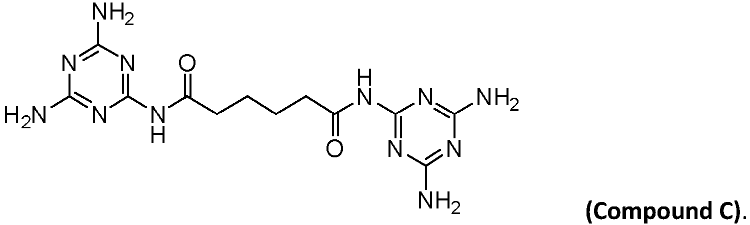

According to a further specific embodiment, the invention provides a non-halogenated melamine compound for use as a flame retardant with the aforesaid TGA characteristics, wherein A is absent, Y is 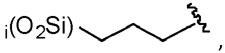 X is melamine, and n is 1, such that the compound has the structure below:

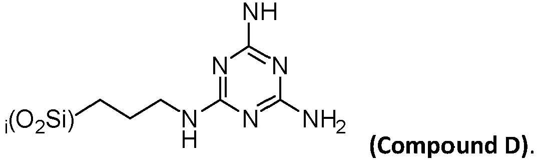

According to yet another specific embodiment, the invention provides a non-halogenated melamine compound for use as a flame retardant with the aforesaid TGA characteristics, wherein A is absent, Y is 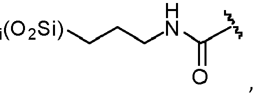 X is a melamine moiety, and n is 1, such that the compound has the structure below (compound L):

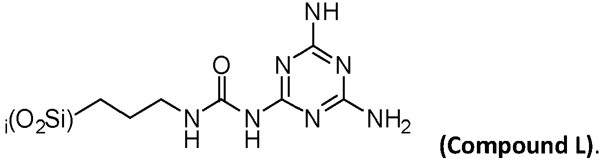

According to a further specific embodiment, the invention provides a non-halogenated melamine compound for use as a flame retardant with the aforesaid TGA characteristics, wherein A is 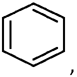 Y is C=O, X is a melamine moiety, and n is 3, such that the compound has the structure below (compound E):

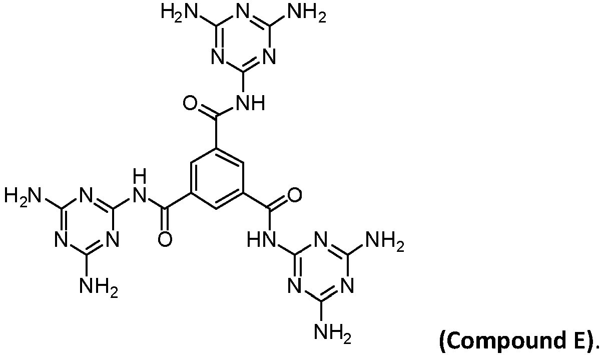

According to a further specific embodiment, the invention provides a non-halogenated melamine compound for use as a flame retardant with the aforesaid TGA characteristics, wherein A is 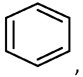 Y is C=O, X is Xₘ, wherein Xₘ is a branched repeating unit of a melamine moiety having a structure according to formula (II), m is an integer from 1-500 and n is 3, such that the compound has the structure below (compound I): 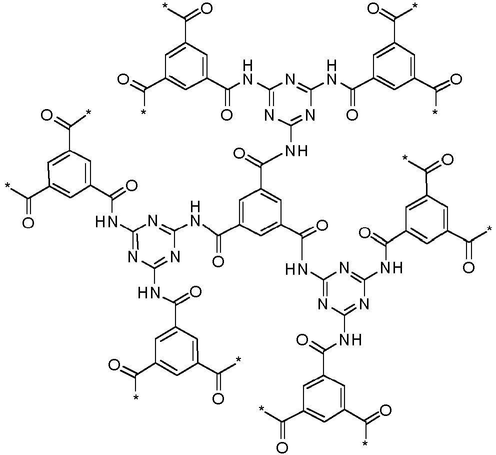 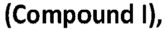 wherein the asterisk (*) denotes the point of attachment in the repeating unit to which additional X groups having a structure according to formula (II) are added.

According to a further specific embodiment, the invention provides a non-halogenated melamine compound for use as a flame retardant with the aforesaid TGA characteristics, wherein A and Y are absent, X is Xₘ, wherein Xₘ is a branched repeating unit of melamine having a structure according to formula (III), and m is an integer from 1-500 such that the compound has the structure below: 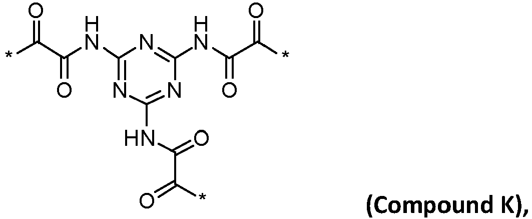 wherein the asterisk (*) denotes the point of attachment in the repeating unit to which additional X groups having a structure according to formula (III) are added.

According to yet another specific embodiment, the invention provides a non-halogenated melamine compound for use as a flame retardant with the aforesaid TGA characteristics, wherein A is 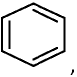 Y is C=O, X is a melamine moiety and n is 6, such that the compound has the structure below (compound F):

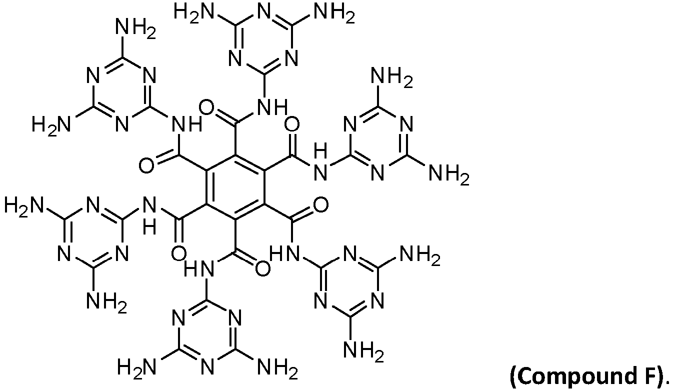

According to anembodiment, the invention provides a non-halogenated melamine compound for use as a flame retardant with the aforesaid TGA characteristics, where any one of the aforementioned compounds is a salt.

In an embodiment, said salt comprises a positively charged melamine cation.

In an embodiment, said salt comprises a negatively charged phosphorous-containing anion.

In an embodiment, said negatively charged phosphorous-containing anion is selected from phosphate, polyphosphate and phenylphosphonate.

According to another specific embodiment, the invention provides a non-halogenated melamine compound for use as a flame retardant with the aforesaid TGA characteristics, wherein the compound is a phosphate salt of aforementioned compound J such that the salt has the structure below (Compound J-S1):

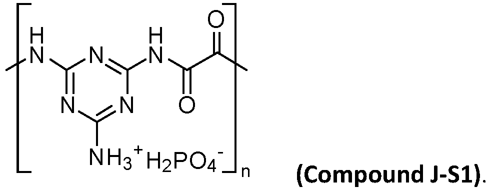

According to another specific embodiment, the invention provides a non-halogenated melamine compound for use as a flame retardant with the aforesaid TGA characteristics, wherein the compound is a polyphosphate salt of aforementioned compound J such that the salt has the structure below (Compound J-S2):

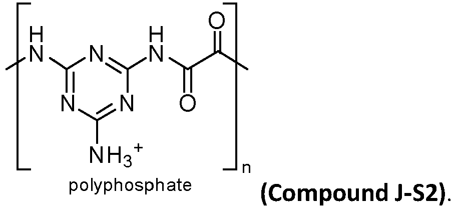

According to another specific aspect there is discussed a non-halogenated melamine compound for use as a flame retardant with the aforesaid TGA characteristics, wherein the compound is a phosphate salt of aforementioned compound O such that the salt has the structure below (Compound O-S):

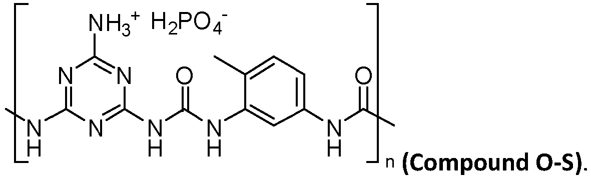

According to another specific aspect there is discussed a non-halogenated melamine compound for use as a flame retardant with the aforesaid TGA characteristics, wherein the compound is a polyphosphate salt of aforementioned compound P such that the salt has the structure below (Compound P-S):

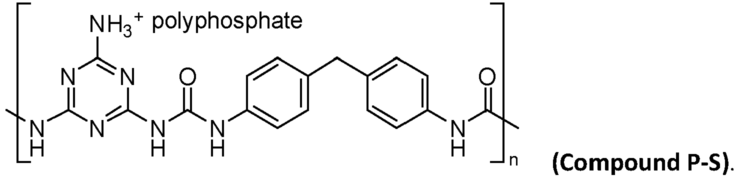

According to another specific embodiment, the invention provides a non-halogenated melamine compound for use as a flame retardant with the aforesaid TGA characteristics, wherein the compound is a polyphosphate salt of aforementioned compound D such that the salt has the structure below (Compound D-S):

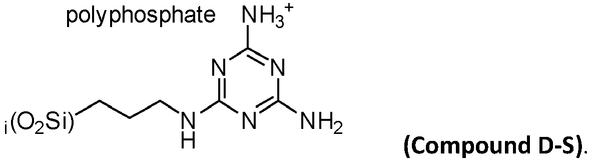

According to another specific embodiment, the invention provides a non-halogenated melamine compound for use as a flame retardant with the aforesaid TGA characteristics, wherein the compound is a polyphosphate salt of aforementioned compound E such that the salt has the structure below (Compound E-S1):

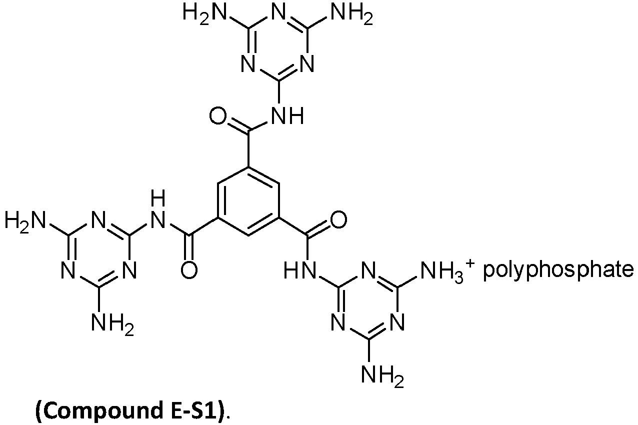

According to another specific embodiment, the invention provides a non-halogenated melamine compound for use as a flame retardant with the aforesaid TGA characteristics, wherein the compound is a phenylphosphonate salt of aforementioned compound E such that the salt has the structure below (Compound E-S2):

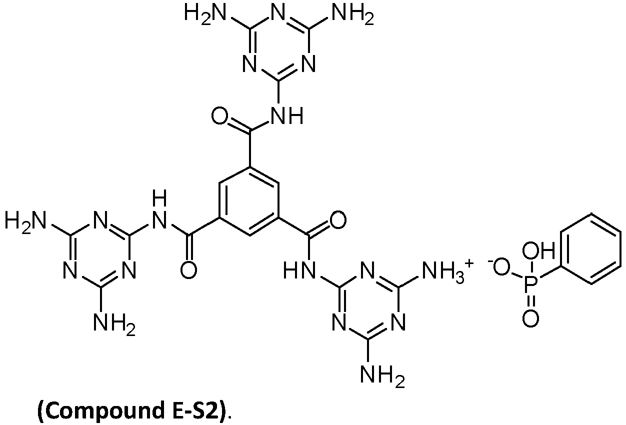

According to another specific aspect there is discussed a non-halogenated melamine compound for use as a flame retardant with the aforesaid TGA characteristics, wherein the compound is a phenylphosphonate salt of aforementioned compound N such that the salt has the structure below (Compound N-S):

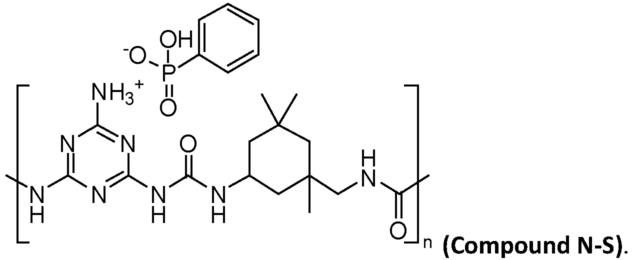

In another aspect, the invention relates to a flame retarded plastic material comprising a compound in accordance with the invention.

In yet another aspect, the invention encompasses an article of manufacture made of, or coated with a compound in accordance with the invention.

In one embodiment of the invention, the flame retarded plastic material or the article described above, which may be made of plastic material or of any other material, such as wood, textile, etc., further comprise additives such as synergists, stabilizers, such as tridecylphosphite, barium-cadmium soaps and organotin compounds, inorganic fillers, plasticizers, such a terephthalates, antioxidants, lubricants or colorants.

In yet another aspect, the present invention provides compositions comprising one or more non-halogenated melamine compounds as described above, wherein about 0.1 - 50 percent by weight of the composition consists of said melamine compounds.

In one embodiment of the invention, about 5 - 30 percent by weight of the composition consists of said melamine compounds.

### BRIEF DESCRIPTION OF THE FIGURES

In the figures:

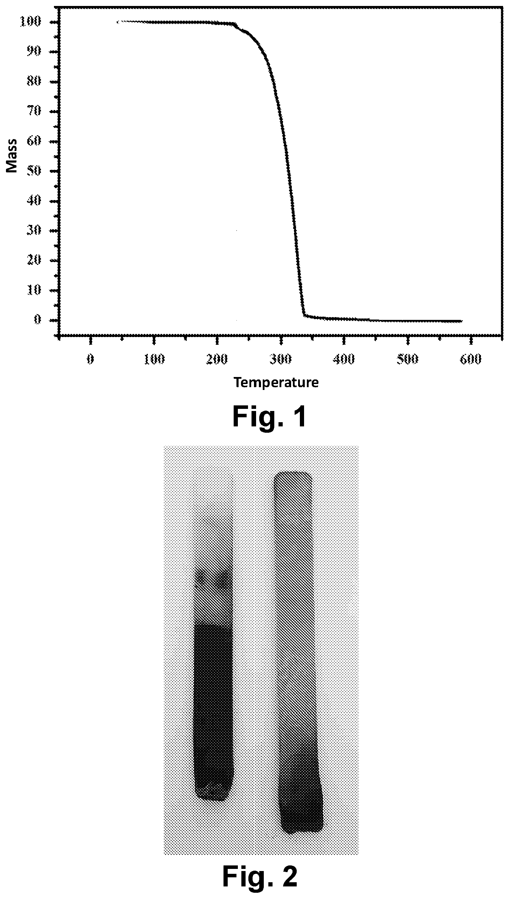

- Fig. 1 shows a TGA plot for a melamine standard acquired while heating the sample from about 50 °C to about 600 °C. Mass is plotted as percent of initial mass (%) and temperature is in degrees Centigrade (°C);
- Fig. 2 shows samples from a UL 94 plastic flammability standard test conducted with a coupon of acrylonitrile butadiene styrene without an additive (left) as a control and acrylonitrile butadiene styrene with an additive (right) which passed the UL 94 test, in which the additive loaded was poly(melamine-co-oxalyl) (compound J);

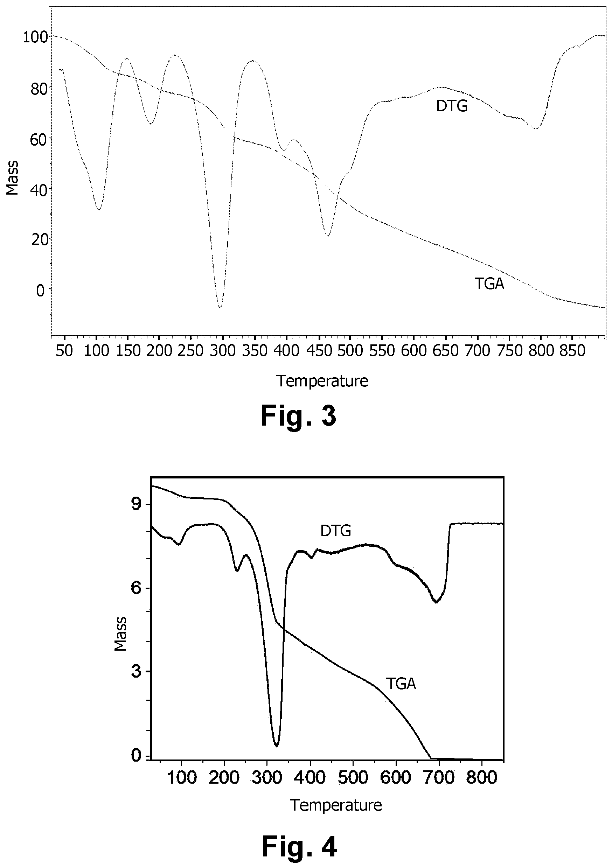

- Fig. 3 shows a TGA plot for compound A (hexakismelamine cyclotriphosphazene amide), acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);

- Fig. 4 shows a TGA plot for compound E (trimelamine benzene tricarboxamide), acquired while heating the sample from 30°C - 900 °C at 10 °C/min under a flow rate of 60mL/min, overlaid with the DTG plot derived therefrom, wherein each curved is labeled accordingly. Mass is plotted in milligrams (mg) and temperature is in degrees Centigrade (°C);

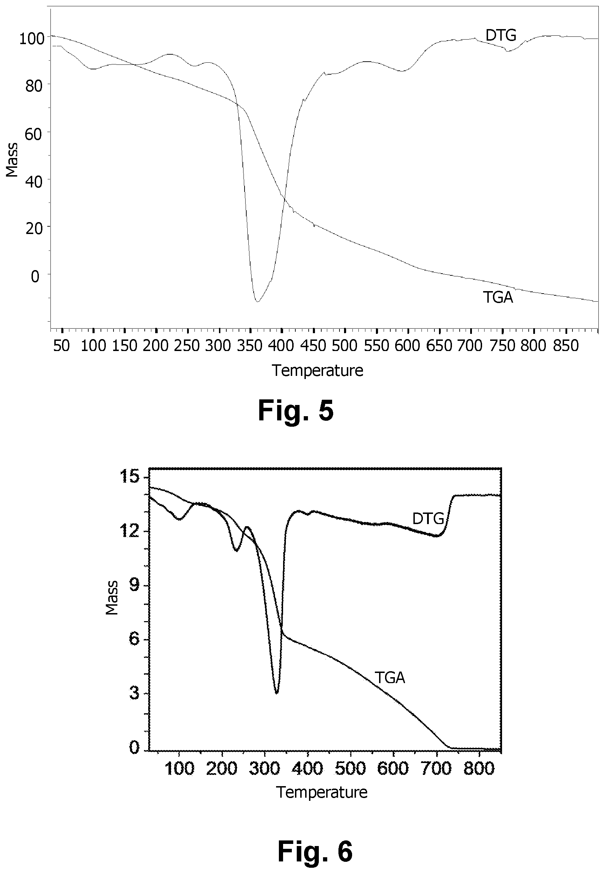

- Fig. 5 shows a TGA plot for compound I (poly(melamine-co-benzene tricarboxamide), acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);
- Fig. 6 shows a TGA plot for compound F (hexakis melamine benzene hexacarboxamide), acquired while heating the sample from 30°C - 900 °C at 10 °C/min under a flow rate of 60mL/min, overlaid with the DTG plot derived therefrom, wherein each curved is labeled accordingly. Mass is plotted milligrams (mg) and temperature is in degrees Centigrade (°C);

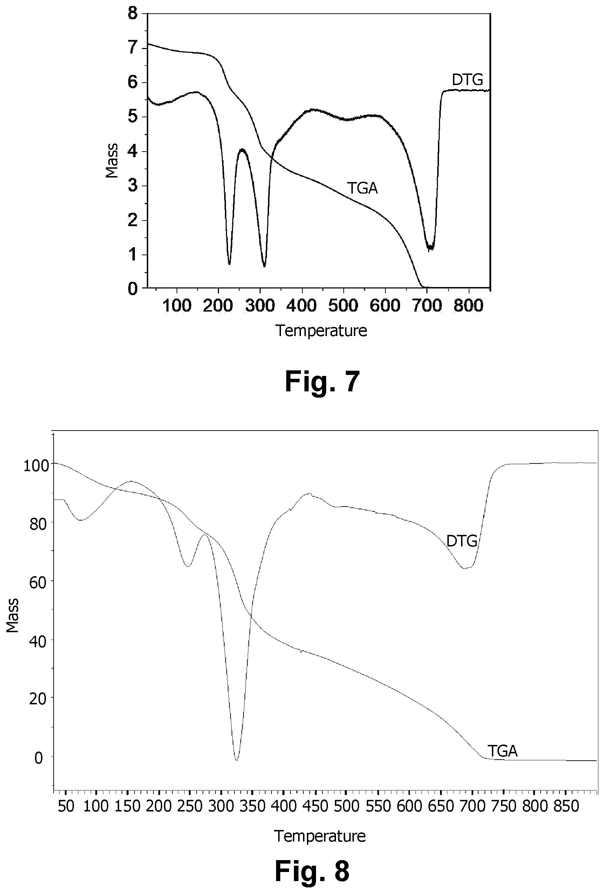

- Fig. 7 shows a TGA plot for compound B (polyurea-melamine), acquired while heating the sample from 30 °C - 900 °C at 10 °C/min under a flow rate of 60mL/min, overlaid with the DTG plot derived therefrom, wherein each curved is labeled accordingly. Mass is plotted in milligrams (mg) and temperature is in degrees Centigrade (°C);
- Fig. 8 shows a TGA plot for compound J (poly(melamine-co-oxalyl), afforded via a 3-step synthesis, acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);

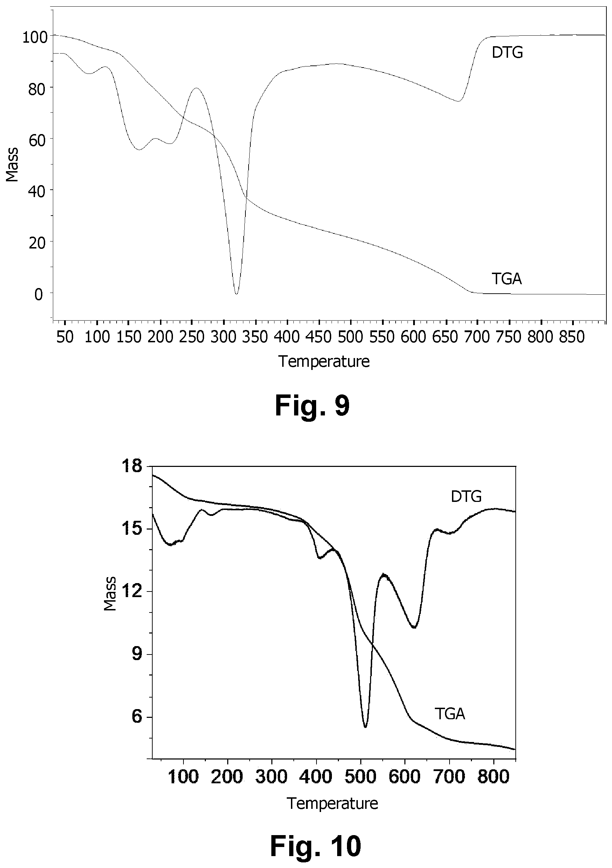

- Fig. 9 shows a TGA plot for compound K (3D poly(melamine-co-oxalyl)) acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);
- Fig. 10 shows a TGA plot for compound D (3-melaminepropyl silica), acquired while heating the sample from 30 °C - 900 °C at 10 °C/min under a flow rate of 60mL/min, overlaid with the DTG plot derived therefrom, wherein each curved is labeled accordingly. Mass is plotted in milligrams (mg) and temperature is in degrees Centigrade (°C);

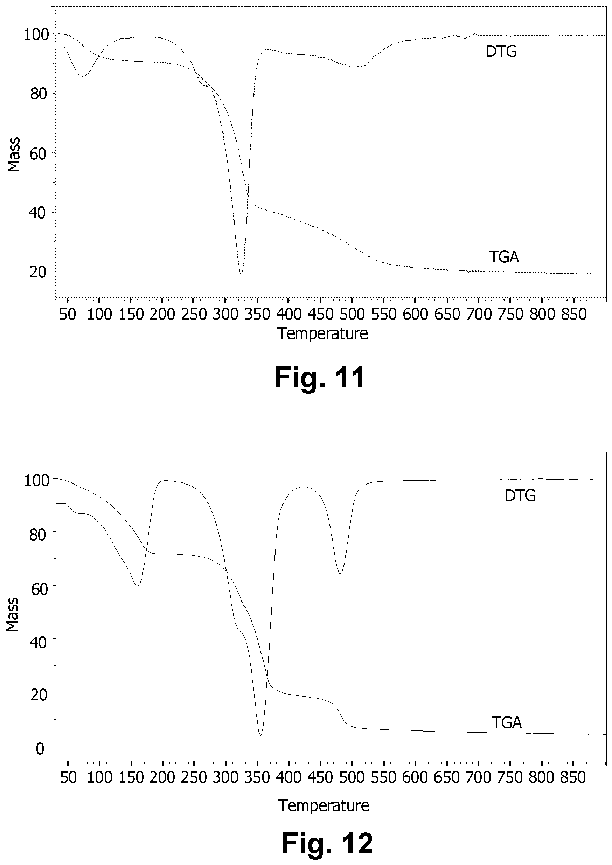

- Fig. 11 shows a TGA plot for compound L (3-melamineureapropyl silica) acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);
- Fig. 12 shows a TGA plot for compound M (poly(melamine-co-hexamethylene urea)) acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);

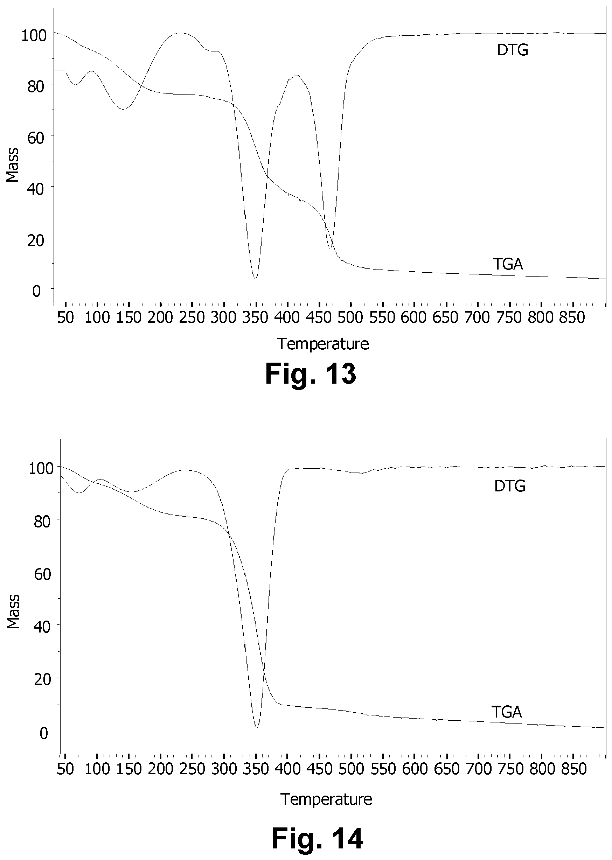

- Fig. 13 shows a TGA plot for compound N (poly(melamine-co-isophorone urea)) acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);
- Fig. 14 shows a TGA plot for compound O (poly(melamine-co-tolyl urea)) acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);

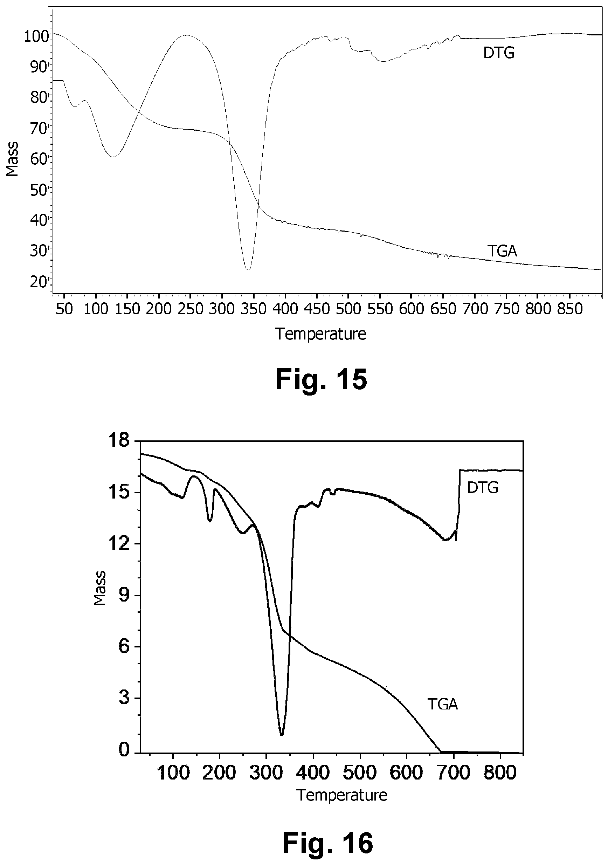

- Fig. 15 shows a TGA plot for compound P (poly(melamine-co-bisphenylmethylene urea)) acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);
- Fig. 16 shows a TGA plot for compound C (bismelamine adipoyl diamide), acquired while heating the sample from 30 °C - 900 °C at 10 °C/min under a flow rate of 60mL/min, overlaid with the DTG plot derived therefrom, wherein each curved is labeled accordingly. Mass is plotted in milligrams (mg) and temperature is in degrees Centigrade (°C);

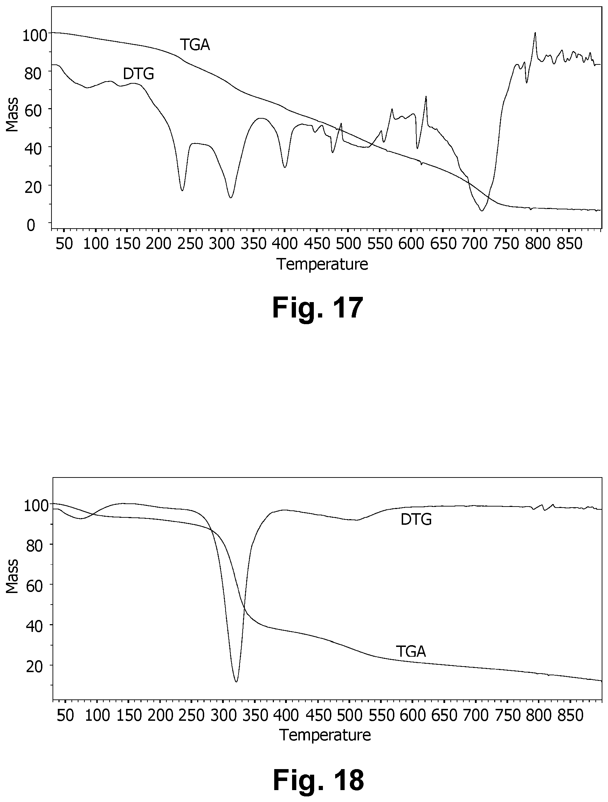

- Fig. 17 shows a TGA plot for compound J-S1 (poly(melamine-co-oxalyl phosphate salt), acquired while heating the sample from 30°C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);
- Fig. 18 shows a TGA plot for compound O-S (poly(melamine-co-tolyl urea) phosphate salt), acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);

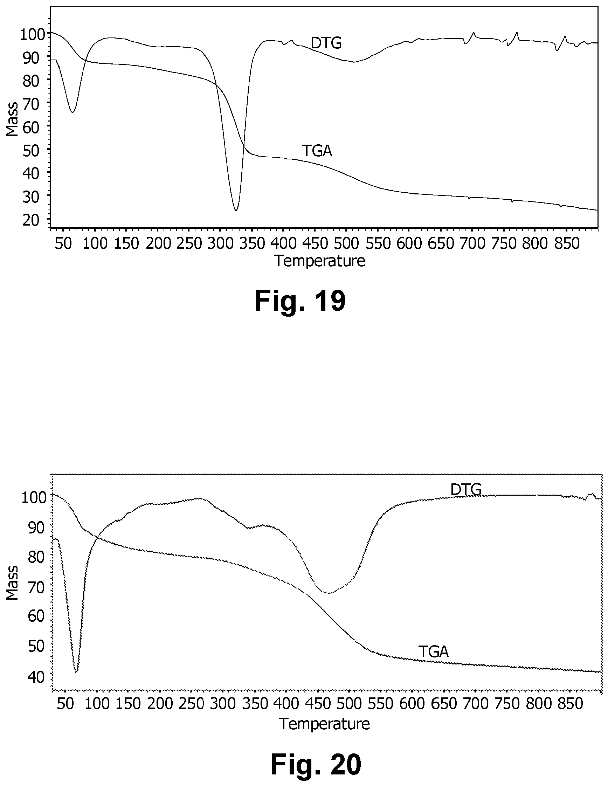

- Fig. 19 shows a TGA plot for compound P-S (poly(melamine-co-bisphenylmethylene urea) polyphosphate salt), acquired while heating the sample from 30°C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);
- Fig. 20 shows a TGA plot for compound D-S (3-melaminepropyl silica polyphosphate salt), acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);

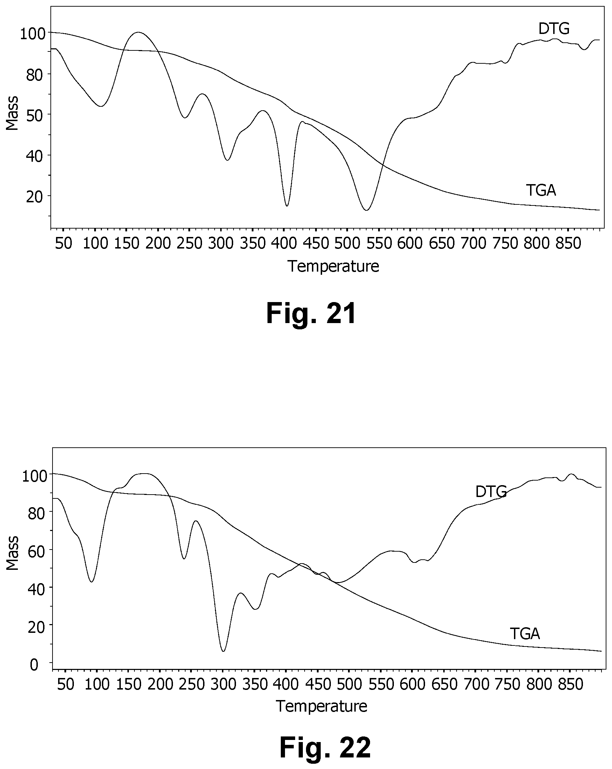

- Fig. 21 shows a TGA plot for compound E-S1 (trimelamine benzene tricarboxamide polyphosphate salt), acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);
- Fig. 22 shows a TGA plot for compound E-S2 (trimelamine benzene tricarboxamide phenylphosphonate salt), acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C);

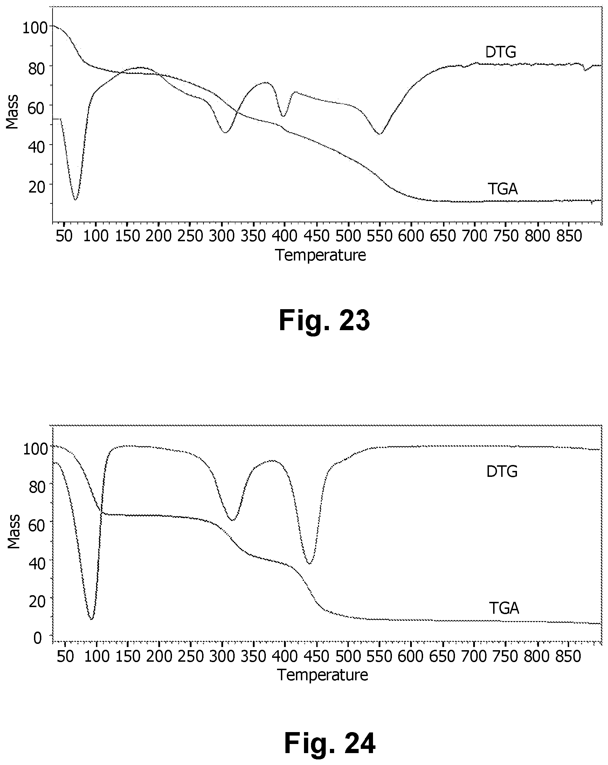

- Fig. 23 shows a TGA plot for compound J-S2 (poly(melamine-co-oxalyl polyphosphate salt), acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C); and
- Fig. 24 shows a TGA plot for compound N-S (poly(melamine-co-isophorone urea) phenylphosphonate salt), acquired while heating the sample from 30 °C - 900 °C at 10 °C/min under a flow rate of 60mL/min, overlaid with the DTG plot derived therefrom, wherein each curved is labeled accordingly. Mass is plotted as percent of initial mass (%) and temperature is in degrees Centigrade (°C).

### DETAILED DESCRIPTION OF THE INVENTION

The compounds of the present invention address the need for improved and environmentally-friendly flame-retarding materials. The compounds disclosed herein exhibit highly desirable decomposition characteristics, as will be detailed further herein, are devoid of halogens, which obviate halogen-related health concerns, and the majority is afforded by incorporating a few melamine units in novel chemical configurations. Furthermore, it will be apparent to those of skill in the art that the flame retardant compounds disclosed by the present invention can easily be used in a variety of applications, such as, but not limited to those which presently employ halogenated flame retardants thus replacing halogen-containing flame retardants, which, in many applications, consist of either brominated or chlorinated compounds which may pose a serious risk to individuals and potential harm to the environment.

It will also be evident to the skilled person that the melamine compounds of the invention may suitably be added to any compatible resin, polymer, compositions, or any suitable material, regardless of whether the substance is flammable or not, in amounts sufficient to give the desired degree of flame retardancy. Indeed, the skilled worker appreciates that the amount required to give a desirable flame retardant polymer varies widely depending upon the particular polymer, the shape of the polymer in the final form and the level of flame retardancy desired. Thus, a polymer or a composition comprising one or more melamine compounds of the present invention would broadly contain a flame retarding amount of the compounds of the invention as readily understood by the skilled worker. Accordingly, a flame retarding amount as referred to herein is to be understood as an amount of the flame retarding compound of the invention which when present in the polymer, or any suitable material, measurably reduces the tendency of the polymer, or any suitable material, to thermally decompose, burn or to ignite.

In some embodiments, the invention discloses compositions comprising one or more melamine compounds of the invention, wherein about 0.1 - 50 percent by weight of the composition corresponds to melamine compounds of the invention, for example, compositions containing about 5 - 30 percent by weight of the melamine compounds of the invention. The compositions comprising one or more melamine compounds of the invention have excellent flame retardancy which can be obtained while the desirable properties of the polymers are substantially retained.

As the skilled person will surely recognize, even within these ranges and preferred ranges, the particular concentrations and ranges used in a particular mixture will vary as aforesaid above and will depend on the amount of flame retardancy desired at the concessions to other properties of the composition, such as thermal stability, color, toxicity and odor, that can be yielded. The determination of these ranges with the above in mind can be easily obtained by those of skill in the art especially with the aid of the well-known teachings of the art.

It should be noted that any material such as resin or a composition with which a compound of the invention is compatible may be made flame resistant by any number of methods that are well-known in the art such as, but not limited to physically mixing said compatible material with a compound of the present invention during, for example, the manufacturing process, e.g. thermosetting, etc., to obtain a product which is resistant to decomposing and/or burning while at the same time substantially retaining the desirable properties of the untreated material. Such materials include but are not limited to resins containing rubber, polyethylene, polypropylene, polystyrene, polyimide, polyamide, polyester, polyurethane, polycarbonate, acrylates, urea formaldehyde, and polysulfones, acrylic, butyl, cellulosics, epoxy, furan, neoprene, nitrile, nitrocellulose, phenolic, polyamide, poly ester, polyether, polyolefin, polysulfide, polyurethane, poly vinyl butyral, silicone, styrene-butadiene, butyl rubber, and vinyl, which may be in any form such as liquids, solids, fibers and the like.

More specifically, polymer and polymer compositions to which the flame retardants of the invention are applicable include (but are not limited to) the following categories and members therein:
1. Mono- and di-olefins such as polypropylene (PP), thermoplastic olefins (TPO), polyisobutylene, polymethylpentene, polyisoprene, polybutadiene, polyethylene with or without cross-linking, high-density polyethylene, low-density polyethylene, or mixtures of these polymers. Copolymers of mono and di olefins including other vinyl monomers such as ethylene-propylene copolymers, ethylene-vinyl acetate copolymers. Terpolymers of ethylene with propylene and a diene such as hexadiene, cyclopentadiene or ethylidiene norborene and vinyl monomers such as vinyl acetate. Mixtures of polymers under 1.
2. Polystyrene, poly p-methyl styrene, poly alpha- methylstyrene, and copolymers of styrene or alpha-methylstyrene with dienes or acryl derivatives such as styrene-butadiene, styrene-actrylonitrile, styrene-alkylmethylacrylate, styrene-butadiene-akylacrylate, styrene-maleic anhydride, and styrene-acrylonitrile-methylacrylate.
3. Polyphenylene oxide and polyphenylene sulfide and their mixtures with styrene polymers or with polyamides.
4. Polyurethanes derived from polyethers, polyesters and polybutadiene with terminal hydroxy groups on one hand and aliphatic or aromatic polyisocyanates on the other as well as their precursors.
5. Polyamides and copolymers derived from diamines and dicarboxylic acids and/or from aminocarboxylic acids or the corresponding lactams, such as polyamide 4, polyamide 6, polyamide 6/6, 6/10, 6/12, 4/6, 66/6, 6/66, polyamide 11, polyamide 12, aromatic polyamides based on aromatic diamine and adipic acid: and iso- and/or terephthalic acid and optionally an elastomer as modifier, for example poly-2,4-trimethyl hexamethylene terephthalamide, poly m phenylene-isophthalamide.
6. Polyesters derived from dicarboxylic acids and dialcohols and/or from hydrocarboxylic acids or the corresponding lactones such as polyethylene terephthalate, polybutylene terephthalate, polyethylene terephthalate/ polybutylene terephthalate mixtures, polyethylene terephthalate/polybutylene terephthalate copolymers, poly 1,4-dimethyl ciclohexane terephthalate, polyhydroxybenzoates, and co-polymers with ethylene.
7. Polyvinyl chloride and copolymers with ethylene, copolymers of tetra fluro ethylene and ethylene.
8. Thermoset polymers include for example unsaturated polyester resins, saturated polyesters, alkyd resins, amino resins, phenol resins, epoxy resins, diallyl phthalate resins, as well as polyacrylates and polyethers containing one or more of these polymers and a cross linking agent. A review of thermosets is found in Ullmann's Encyclopedia of Industrial Chemistry, Vol A26, p 665
9. Polymers for insulation such as fluorinated ethylene-propylene (FEP), cross linked polyethylene (XLPE), ethylene-propylene rubber (EPR), tree cross linked polyethylene (TRXLPE), and ethylene vinyl acetate (EVA).
10. Cellulose acetate, flexible polyurethane, rigid polyurethane.
11. Fluoropolymers and co-polymers such as TEFZEL^{®}, DuPont Co, Wilmington, Del. Elastomers such as spandex as defined in Encyclopedia of Chemical Technology. Polyimides such as KAPTON^{®}, DuPont Co., Wilmington, Del. And defined in Encyclopedia of Chemical Technology.
12. Polyethylene and its co-polymers.
13. Ethylene vinyl acetate, ethylene vinyl acetate carbon monoxide and ethylene n butyl acrylate carbon monoxide and ethylene n butyl acrylate glycidyl methacrylate, ethylene methyl, ethyl, and butyl acrylate ethylene (methyl, ethyl, buthyl) acrylate-vinyltrimethylsilane, or vinyltriethylsilane ethylene methyl acrylate and ethylene methyl acrylate MAME, ethylene acrylic and methacrylic acid, ethylene acrylic and methacrylic acid ionomers (Zn, Na, Li, Mg), maleic anhydride grafted polymers.

Furthermore, one may also incorporate and combine into said resin, polymer or composition, in addition to one or more flame retardant compounds of the invention, other additives such as synergists, stabilizers, such as tridecylphosphite, barium-cadmium soaps and organotin compounds, inorganic fillers, plasticizers, such a terephthalates, antioxidants, lubricants or colorants. Importantly, the flame retarding compounds of the invention, when combined with the above additives ought to impart an effective flame-retarding property without deteriorating the appearance and properties of the final product produced therefrom.

The non-halogenated melamine compounds of the invention can be converted into various non-halogenated salts by reaction with the corresponding acid in order to further augment their thermal properties. In particular embodiments, the compounds of the invention are ionized with various phosphorous containing acids to produce phosphorous-containing melamine salts with improved or extended heat stabilities. These include, among others: phosphates, polyphosphates of various degree and geometry of polymerization and various phosphonates such as phenylphosphonate esters.

The term "polyphosphate" encompasses both salts and esters formed from the condensation of phosphate units. As such, polyphosphates can adopt linear or a cyclic ring structure. Furthermore, high molecular weight polyphosphates such as the glassy (i.e., amorphous) Graham's salt., and crystalline high molecular weight polyphosphates such as Kurrol's salt and Maddrell's salt also fall under the category of polyphosphates as defined herein.

In instances wherein foamed articles of suitable polymers are desired, a blend of the aforementioned ingredients and a blowing agent, preferably a solid material, which blend is stable at temperatures used for blending the ingredients, is prepared in a manner to provide a uniform dispersion of the flame retardant additive and the blowing agent in the polymer. The resulting composition may then be molded in a form such as a cavity mold at temperatures sufficient to activate the blowing agent. Other conventional techniques for making foamed polymers are also suitably employed. It should also be noted that although stabilizers are generally unnecessary with the pure flame retardants of the invention, commercial grades of the flame retardant additives may sometimes contain impurities which cause discoloration at high temperatures. With proper stabilization or purified additives, however, polystyrene rendered flame retardant by the compounds of the invention, for example, may be molded or extruded without degradation or discoloration.

In addition to the foregoing, the skilled artisan will appreciate that the compounds of the invention are effective flame retardants when used alone in any manufacture application, but they are compatible with and may synergize with other known additives, such as other flame retardants, which may or may not have been explicitly mentioned herein, but are nonetheless well known to the skilled artisan, and may be used in combination with the compounds of the invention.

It should be apparent that the compounds of the invention may be employed satisfactorily as flame retardants in a variety of applications to produce articles such as but not limited to plastics, paper, paints, textiles, electronics, upholstered furniture, and building products like insulation.

Furthermore, any suitable polymer or composition containing a flame-retardant melamine compound of the invention can be made to show any one or more of the following advantageous features:
1. a high flame-retarding effect obtained with even a small amount of the flame retardant;
2. the favorable properties inherent to the polymer are little affected by the flame-retardant because a relatively small amount of the retardant is required;
3. substantially no stain is imparted to the finished product in the process;
4. substantially no foaming takes place in a molding process; and
5. substantially no bleed-out of the flame retardant takes place during or after the process.

To define more clearly the terms used herein, the following definitions are provided below. To the extent that any definition or usage known from the art conflicts with the definition or usage provided further herein, the definition or usage provided further herein prevails.

The phrase "a" or "an" entity as used herein refers to one or more of that entity; for example, a compound refers to one or more compounds or at least one compound. As such, the terms "a" (or "an"), "one or more", and "at least one" can be used interchangeably herein.

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components.

As used herein, unless specifically indicated otherwise, the word "or" is used in the "inclusive" sense of "and/or" and not the "exclusive" sense of "either/or".

The term "independently" is used herein to indicate that a variable is applied in any one instance without regard to the presence or absence of a variable having that same or a different definition within the same compound. For example, in a compound in which R¹ appears twice and is defined as "independently carbon or nitrogen", then both R¹ can be carbon, both R¹ can be nitrogen, or one R¹ can be carbon and the other nitrogen.

When any variable occurs more than one time in any moiety or formula depicting and describing compounds employed or claimed in the present invention, its definition on each occurrence is independent of its definition at every other occurrence. Also, combinations of substituents and/or variables are permissible only if such compounds result in stable compounds.

The term "optional" or "optionally" as used herein means that a subsequently described event or circumstance may, but need not, occur, and that the description includes instances where the event or circumstance occurs and instances in which it does not. For example, "optionally substituted" means that the optionally substituted moiety may incorporate a hydrogen atom or a substituent.

The terms "about" and "substantially" as used herein mean that amounts, sizes, formulations, parameters, and other quantities and characteristics are not and need not be exact, but may be approximate and/or larger or smaller, as desired, reflecting tolerances, conversion factors, rounding off, measurement errors, and the like, and other factors known to those of skill for which their description by such terms is widely accepted in the art. In general, an amount, size, formulation, parameter or other quantity or characteristic is "about" or "approximate" whether or not expressly stated to be such. The term "about" also encompasses amounts that may differ due to different equilibrium concentrations of components in a composition resulting from a particular initial mixture. Whether or not modified by the term "about", the claims include such equivalents to the quantities recited therein. In the context of experimental measurements, the term "about" can mean within 10% of the reported numerical value, preferably within 5 % of the reported numerical value.

The term "thermogravimetric analysis" (TGA) with respect to a flame-retardant compound, as used herein, refers to a quantitative measurement of substance's thermal stability that is performed on a thermogravimetric analyzer, as is known to those of skill in the art. Briefly, in TGA, the mass of a sample is measured over time as the temperature is varied. The output is the mass remaining, typically as a percentage of the initial mass, (plotted on the vertical axis) as a function of the temperature at which the remaining mass was recorded (plotted on the horizontal axis).

The term "onset decomposition temperature" or "ODT", as used herein, is defined as the temperature at which an appreciable loss in mass of a substance is first recorded, which can be determined accurately using the first derivative of the TGA curve (i.e., the derivative thermogravimetric curve or DTG for short).

Further, when plotted, the DTG curve can be used to determine the locations of heat-labile regions which are ranges of temperature where inflections can be seen in the TGA curve (i.e., temperatures where the change in mass of a substance is large), and heat-stable regions, also referred to as stability zones, which are ranges of temperature where the slope of the TGA curve is close to zero (i.e., temperatures where the change in mass of a substance is small).

Accordingly, as the start and end positions of stability zones are flanked by inflection points in the TGA curve, temperature ranges at which a compound would be deemed as having "heat stability" or "thermal stability" could be easily discerned.

Conversely, the term "thermal stability" or "heat stability" as used herein refers specifically to traits observed in the TGA plot of a compound. In particular, a compound is said to exhibit thermal stability across a range of temperatures through which little to no loss in its mass is recorded, as determined by the DTG plot. Similarly, when a compound is described or defined as "exhibiting thermal stability *up to* a certain temperature", or an expression of the like, this is to be interpreted as stating that said compound features relatively small losses in its mass as the temperature varies within said range (although the cumulative mass loss may be significant) up to approximately said certain temperature at which point the compound is said to have thermally decompose. It should however be noted that according to the above definition, a compound that exhibits thermal stability across a certain temperature range does not necessarily remain in the same chemical and/or physical state across said certain temperature range, and hence may still undergo a chemical reaction and/or physical change within its stability zone.

On the other hand, TGA may be useful for in-depth interpretation of the decomposition reaction (e.g., combustion, pyrolysis, evaporation, etc.). For example, melamine compounds and their derivatives may exhibit an onset decomposition temperature or a heat-labile region around 300-350 °C, and DTG features corresponding to these temperature ranges are sometimes attributed to the decomposition of the melamine group in said compounds. Nevertheless, the interpretation of the chemistry of the decomposition and other TGA results herein is qualitative and does not necessarily bind the invention, which rests on its own distinct properties, to one mechanism or another.

As stated above, it was surprisingly found that novel non-halogenated melamine compounds as disclosed herein exhibit unexpected and highly useful flame retardant characteristics. This is illustrated, for example, by comparing the TGA curves in Figs. 2 and 1. As can be seen in Fig. 1, melamine by itself exhibits a single inflection point in its TGA curve occurring at about 325 °C, which in turn limits its efficiency as a flame retardant above this temperature. In contrast, Fig. 2 show that compound A, which incorporates six melamine moieties substituted unto a tricyclophosphazene core, exhibits multiple, spread out, overlapping inflection points in its TGA curve, some of which occurr at about 100, 180, 300 400 and 450 °C. Thus, compound A, as well as the remaining compounds of the invention, will retard flames above 300 °C far more efficiently than melamine alone, and hence, further extend the utility of the novel non-halogenated melamine compounds to applications requiring flame resistance at diverse and/or extended temperatures.

Consistent with the aspects of the present invention, the terms "TGA₂₅", "TGA₅₀", and "TGA₇₅", as used herein, are parameters that characterize TGA plots and are defined as the temperatures at which 25%, 50% and 75% of the sample's initial mass has been lost in a TGA experiment, respectively. It will be apparent to those skilled in the art that said TGA parameters, which may be derived directly from a TGA curve (with the optional aid from the DTG curve) of a flame retardant compound, are defined relative to the heating and air flow rates employed during the experiment as well as with regard to the atmosphere in which the experiment is performed. While not being limited, the TGA₂₅, TGA₅₀, and TGA₇₅ parameters reported herein may be measured relative to a heating rate of 10 °C/min and an air flow rate of 60 mL/min wherein the experiment is conducted under nitrogen or under atmospheric conditions.

Unless otherwise specified, all percentages disclosed herein are percentages by weight (w/w); all temperatures are in degrees Centigrade (°C) and each thermogravimetric analysis is performed with a flow of nitrogen or air at 60 ml per minute and at a heating rate of 10 °C per minute.

The following definitions apply to the chemical structures defined herein:
The term "small molecule" is used herein to refer to any compound lacking a repeating chemical unit, and having a molecular weight less than or equal to 2000 Daltons.

The term "polymer" or any expression that includes said term is used to refer to any compound which possess a repeating chemical unit regardless of the size of said compound.

If a substituent is designated to be "absent", the substituent is not present.

The term "n-butyl" as used herein denotes a radical of an unbranched saturated hydrocarbon chain containing 4 carbon atoms.

The term "C=O" as used herein denotes a carbonyl functional group.

The term (SiO₂)_{I} does not denote a specific species of silica but rather refers to the bulk phase of silica.

The term "amino" as used herein denotes a group of the formula -NR'R" wherein R' and R" may be independently hydrogen, C=O or melamine.

The term "alkyl" as used herein denotes a saturated group of the formula -CₙH₂ₙ₊₁ or -CₙH₂ₙ-, where n represents a number or integer between 1 and 6.

The term "linker" as used herein denotes any group such as, for example, triethylene glycol (also abbreviated as PEG₃) having the linear chemical formula: HOCH₂CH₂OCH₂CH₂OCH₂CH₂OH, which covalently connects two monomeric units in a compound or polymer.

The symbol " 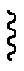 ", which may appear in chemical structures and formulas of compounds and their substituents defined herein, identifies the position in a radical, e.g., a substituent, which attaches to the remaining portion(s) of a molecule as defined in the particular instance or as suggested by the context. For example, in the substituent definition R = 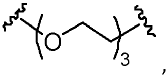 when defined in relation to the formula: AR₂ for example, the " 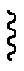 " symbol indicates that substituent "R" is attached via both of its ends, in accordance with other definitions which may be recited of course, to the appropriate groups of "A".

As should be apparent to those of skill in the art, brackets, followed by a subscript or not, may be used in the chemical structures depicted herein to signify that the chemical fragment enclosed by the brackets repeats in a head-to-tail fashion within said compound, where the number of repetitions is indicated by the subscript number if present. Furthermore, it is to be understood that if brackets enclose the entire compound depicted, thereby not designating the terminal groups of the compound, then it is implied that the terminal groups of said compound are selected from any of the moieties appearing within the brackets, supplemented with any necessary hydrogens to complete the structure in a sensible way of course, provided that said terminal groups are logically expected to result when following the synthetic procedures of the instant invention.

The compounds of the invention are defined by TGA curves that are characterized by TGA₂₅, TGA₅₀ and TGA₇₅ parameters, as measured by a thermogravimetric analyzer at a heating rate of 10 °C per minute and at a 60 mL per min air or N₂ flow. Similarly, compounds of the invention possess heat stability regions from about 25 to 1000 degrees Centigrade.

The present invention also describes processes for the preparation of the non-halogenated flame-retardant compounds disclosed herein. The synthetic methods for each compound or group of compounds of the invention are described in detail in the Examples which follow further below.

### EXAMPLES

The invention will now be described with reference to specific examples and materials. The following examples are representative of the techniques employed and data collected by the inventors in carrying out certain aspects of the present invention. It should be appreciated that while the techniques employed are exemplary of the practice of specific embodiments of the invention, those of skill in the art, in light of the present disclosure, will recognize that numerous variations and modifications can be made without departing from the spirit and intended scope of the invention.

### Materials and Methods

Unless indicated otherwise, it should be understood that the compounds of the invention were synthesized from commercially-available starting materials of a purity grade that is suited for synthesis, and isolated from crude reaction mixtures by following appropriate standard work-up protocols and purification procedure that are well-known to those of skill in the field of synthetic chemistry.

### Example 1

### Flame retardant activities of compounds C, D, E and J as determined via Standard Tests for Safety of Flammability of Plastics and Textiles

### Materials:

The following compounds, which were synthesized in accordance with the invention as detailed further herein below, were tested to assess their flame-retardant activities using standard flammability tests: trimelamine benzene tricarboxamide (Compound E, also referred to herein below as "Trimer"), bismelamine adipoylamide (Compound C, also referred to herein below as "adipoyl"), poly(melamine-co-oxalyl) (Compound J, also referred to herein below as "Oxalyl") and 3-melamine propyl silica (Compound D, also referred to herein below as "Silica").

The following standard textiles and polymers were used as substrates onto which the above test compounds were loaded:
Textiles: 100% cotton fabric, or 50%/50% cotton/polyester blend fabric.
Polymers: polypropylene (PP), or acrylonitrile butadiene styrene (ABS).

### Procedures:

### Textiles:

Dispersions were prepared containing the test compounds, a surfactant, a wetting agent and acrylic binder emulsion, all of which are standard components in the following flammability tests. The test fabrics were impregnated with the test dispersion then squeezed to a controlled pick-up of dispersion, followed by drying and curing at 160 °C for 3 minutes. The dry add-on of test material by weight of untreated fabric ranged from 20% to 60%. The test fabrics were tested for flammability according to the ASTM D6413 vertical flammability test.

### Polymers:

Coupons of PP and ABS as per UL 94 were prepared with different loadings of test material. Loadings ranged from 5% to 25% by weight. Samples were tested for flammability according to the UL 94 vertical flammability test.

### Results:

### Textiles:

- Trimer: Flame retardant activity was observed on both 100% cotton and cotton/polyester fabric at add-ons above 50%;
- Adipoyl: Flame retardant activity was observed on both 100% cotton and cotton/polyester fabric at add-ons above 50%;
- Oxalyl: Flame retardant activity was observed on both 100% cotton and cotton/polyester fabric at add-ons above 30%; and
- Silica: Flame retardant activity was observed on both 100% cotton and cotton/polyester fabric at add-ons above 60%.

### Polymers:

- Adipoyl: Limited FR activity on PP; and
- Oxalyl: Limited FR activity on PP, but passed UL 94 at V0 on ABS, as depicted in Fig. 2.

### Example 2

### Preparation of compound A and its thermal stability as determined via TGA

Hexachlorocyclotriphosphazene was used as a starting material to prepare a small molecule with six melamine groups attached to a cyclophosphazene core (compound A). The desired small molecule, also referred to herein as hexakismelamine cyclotriphosphazene amide, was prepared by the rather facile reaction of hexachlorocyclotriphosphazene with six equivalents of melamine in ten equivalents of pyridine in dimethylacetamide as a solvent at 100°C (Scheme 1). To achieve the desired small molecule, a solution of the hexachlorophosphazene was added extremely slowly to a suspension of melamine as an increase in the addition rate resulted in the formation of undesirable cyclophosphazene-melamine polymers.

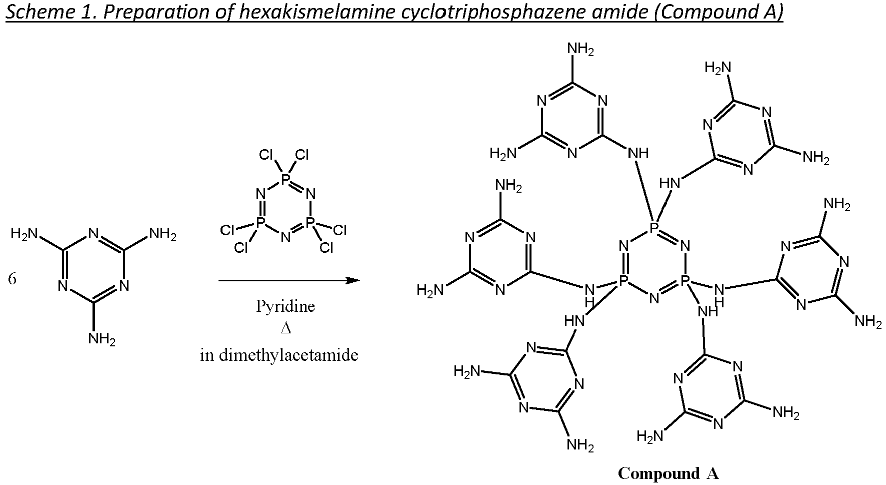

Compound A was isolated and its thermal stability evaluated via TGA, the results of which are shown in Fig. 3. According to the data, compound A exhibited significant thermal stability up to at least790 °C where approximately five overlapping regions of stability spanning this entire temperature range could be observed. Further, compound A showed estimated TGA₂₅, TGA₅₀ and TGA₇₅ values of 250 °C, 410 °C and 560 °C, respectively. Thus, compound A would be recognized by those skilled in the art as an excellent candidate for retarding flames under a similar set of conditions.

### Example 3

### Preparation of compounds B, C, J, 11-J4, and K, and their thermal stabilities as determined via TGA

The general reaction of acid halides with chloromelamines (as described in scheme 2) was utilized in the preparation of a novel family of melamine polymers, containing carbonyl groups as linkers between any two melamine moieties (i.e., the bismelamine adipoyl diamide denoted as compound C, linear polymeric compounds B, J and the homologous series of compounds denoted J1-J4 containing 1-4 intervening methylenes between carbonyl groups, respectively, and the branched polymeric compound K). Triphosgene (a stable substitute to phosgene) or the appropriate diacid halide was reacted with dichloromelamine to form various chain polyamide-melamine polymeric compounds (scheme 2). Likewise, compound C and polymeric compound K were prepared under similar reaction conditions except that monochloromelamine and trichlomelamine, respectively, were used instead of dichloromelamine.

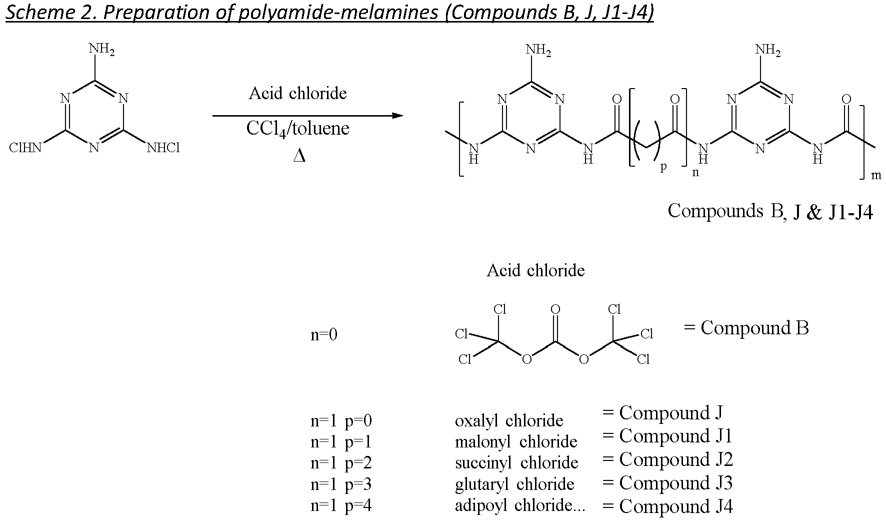

The compounds were isolated and the thermal stabilities of compounds B, C, J and K were evaluated via TGA, the results of which are shown in Figs. 7-9 and 16, and discussed further below.

As shown in Fig. 7, compound B exhibited significant thermal stability up to about 650 °C wherein two local stability zones spanning temperatures of about 50-200 °C and 300-600°C could be observed. Further, compound B showed estimated TGA₂₅, TGA₅₀ and TGA₇₅ values of 275 °C, 375 °C and 600 °C, respectively. Thus, compound B would be recognized by those skilled in the art as a good candidate for retarding flames under a similar set of conditions.

According to Fig. 8, compound J exhibited thermal stability up to at least about 700 °C wherein two local stability zones spanning temperatures of about 50-275 °C and 325-600 °C could be observed. Further, compound J showed estimated TGA₂₅, TGA₅₀ and TGA₇₅ values of 280 °C, 340 °C and 550 °C, respectively. Thus, compound J would be viewed by those skilled in the art as a good candidate for retarding flames under a similar set of conditions.

As shown in Fig. 9, compound K exhibited significant thermal stability up to about 670 °C wherein two local stability zones spanning temperatures of about 30-230 °C and 340-650°C could be observed. Further, compound K showed estimated TGA₂₅, TGA₅₀ and TGA₇₅ values of 200 °C, 310 °C and 440 °C, respectively. Thus, compound K would be recognized by those skilled in the art as a good candidate for retarding flames under a similar set of conditions.

As shown in Fig. 16, compound C exhibited significant thermal stability up to about 650 °C wherein two local stability zones spanning temperatures of about 30-230 °C and 350-650°C could be observed. Further, compound C showed estimated TGA₂₅, TGA₅₀ and TGA₇₅ values of 275 °C, 310 °C and 500 °C, respectively. Thus, compound C would be recognized by those skilled in the art as a good candidate for retarding flames under a similar set of conditions.

### Example 4

### Preparation of compounds D and L, and their thermal stabilities as determined via TGA

These compounds were made based on coupling with commercial functionalized silica. The melamine moiety in the resulting product is covalently bound to the functionalized silica core via an alkyl linker with (scheme 5) or without an intervening urea functional group (scheme 4). These silicone-based melamines (compounds D and L) are suited for incorporation into surfaces (e.g. polymeric sheets, silicone products) or may be used as coatings.

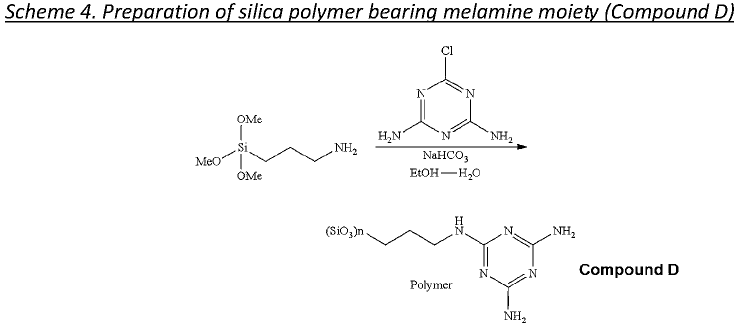

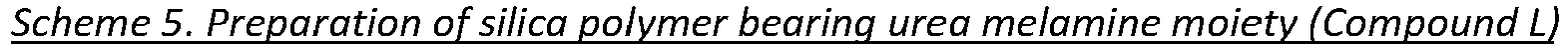

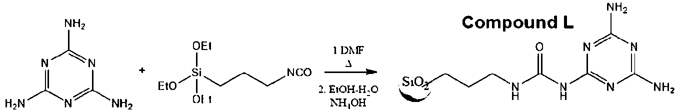

Compounds D and L were isolated and their thermal stabilities evaluated via TGA, the results of which are shown in Figs. 10 and 11, and discussed further below.

As shown in Fig. 10, compound D exhibited significant thermal stability up to about 700 °C. Further, compound D showed estimated TGA₂₅, TGA₅₀ and TGA₇₅ values of 425 °C, 500 °C and 600 °C, respectively. Thus, compound D would be recognized by those skilled in the art as a good candidate for retarding flames under a similar set of conditions.

As shown in Fig. 11, compound L exhibited significant thermal stability up to about 550 °C wherein two local stability zones spanning temperatures of about 100-250 °C and 350-525°C could be observed. Further, compound L showed estimated TGA₂₅, TGA₅₀ and TGA₇₅ values of 300 °C, 330 °C and 520 °C, respectively. Thus, compound L would be recognized by those skilled in the art as a good candidate for retarding flames under similar conditions.

### Example 5

### Preparation of compounds E and I and their thermal stabilities as determined via TGA

A small molecule bearing three melamine moieties (compound E) was prepared from benzene 1,3,5-tricarbonyl trichloride, which was reacted in the usual manner with three equivalents of monochloromelamine (scheme 6). Compound I was synthesized in a similar manner to compound E but where trichloromelamine is used instead of monochloromelamine to afford a 3-D hyperbranched polymer.

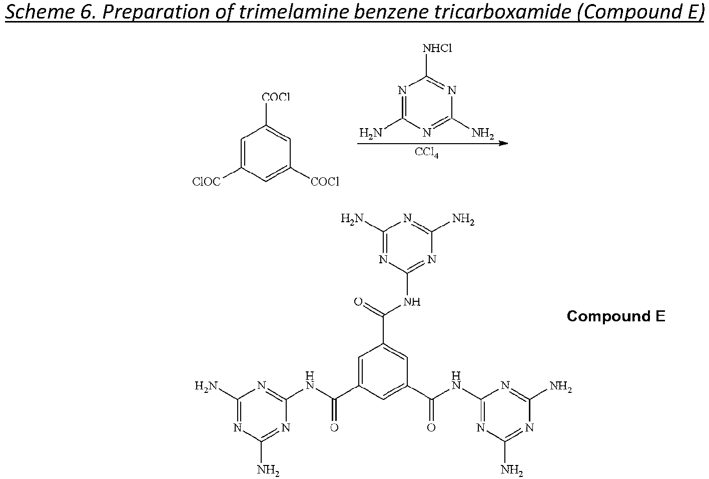

Compounds E and I were isolated and their thermal stabilities evaluated via TGA, the results of which are shown in Figs. 4 and 5, and discussed further below.

As shown in Fig. 4, compound E exhibited significant thermal stability up to a temperature of about 675 °C wherein two local stability zones spanning temperatures 50-250 °C and 350-600 °C could be observed. Further, compound E showed estimated TGA₂₅, TGA₅₀ and TGA₇₅ values of 300 °C, 325 °C and 600 °C, respectively. Thus, compound E would be recognized by those skilled in the art as a good candidate for retarding flames under a similar set of conditions.

As shown in Fig. 5, compound I exhibited significant thermal stability up to at least about 650 °C wherein two local stability zones spanning temperatures of about 50-350 °C and 450-625°C could be observed. Further, compound I showed estimated TGA₂₅, TGA₅₀ and TGA₇₅ values of 290 °C, 370 °C and 420 °C, respectively. Thus, compound I would be recognized by those skilled in the art as a good candidate for retarding flames under similar conditions.

### Example 6

### Preparation of compound F and its thermal stability as determined via TGA

A small molecule bearing six melamine moieties (compound F) was prepared from mellitic acid, which was initially converted into hexa acid chloride with PCI₅, and was then reacted in the usual manner with six equivalents of monochloromelamine (scheme 7).

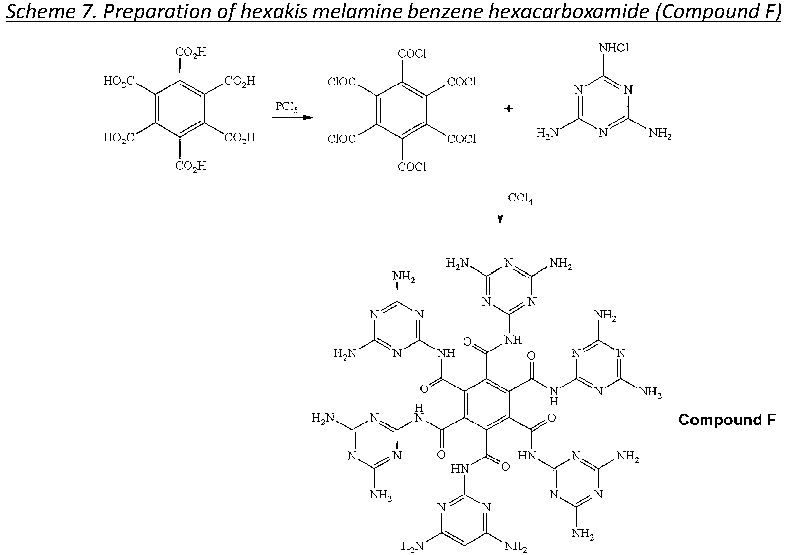

Compound F was isolated and its thermal stability evaluated via TGA, the results of which are shown in Fig. 6. According to the data, compound F exhibited significant thermal stability up to a temperature of about 700 °C wherein two local stability zones spanning temperatures 50-250 °C and 350-600 °C could be observed. Further, compound F showed estimated TGA₂₅, TGA₅₀ and TGA₇₅ values of 300 °C, 325 °C and 550 °C, respectively. Thus, compound F will be recognized by those skilled in the art as a good candidate for retarding flames under a similar set of conditions.

### Example 7 - not according to the invention

### Preparation of compounds M, N, O and P, and their thermal stabilities as determined via TGA

The general reaction of alkylisocynates with melamines in DMF (as described in scheme 8) was utilized in the preparation of a novel family of melamine polymers, containing urea groups as linkers between any two melamine moieties (i.e., compound M, N, O and P).

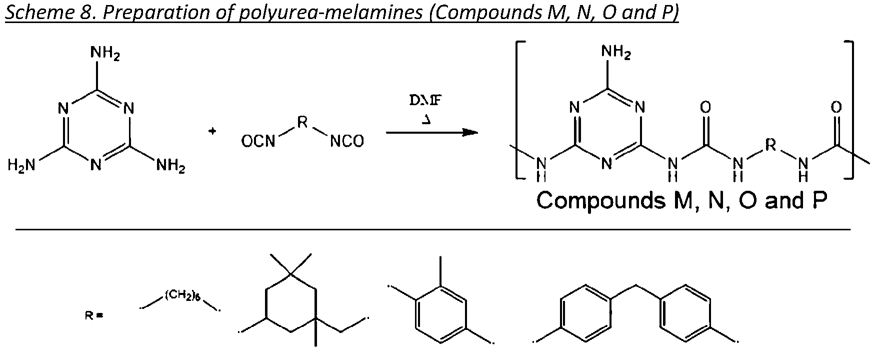

Compounds M, N, O and P were isolated and their thermal stabilities evaluated via TGA, the results of which are shown in Figs. 12-15 and discussed further below.

As shown in Fig. 12, compound M exhibited significant thermal stability up to about 500 °C wherein two local stability zones spanning temperatures of about 175-275 °C and 350-475 °C could be observed. Further, compound M showed estimated TGA₂₅, TGA₅₀ and TGA₇₅ values of 275 °C, 330 °C and 360 °C, respectively. Thus, compound M would be viewed by those skilled in the art as a good candidate for retarding flames under a similar set of conditions.

As shown in Fig. 13, compound N exhibited significant thermal stability up to about 480 °C wherein three local stability zones spanning temperatures of about 70-120 °C, 175-325 °C and 370-450 °C could be observed. Further, compound N showed estimated TGA₂₅, TGA₅₀ and TGA₇₅ values of 280 °C, 350 °C and 460 °C, respectively. Thus, compound N would be viewed by those skilled in the art as a good candidate for retarding flames under a similar set of conditions.

As shown in Fig. 14, compound O exhibited significant thermal stability up to about 370 °C. Further, compound O showed estimated TGA₂₅, TGA₅₀ and TGA₇₅ values of 300 °C, 330 °C and 360 °C, respectively. Thus, compound O would be viewed by those skilled in the art as a good candidate for retarding flames under a similar set of conditions.

As shown in Fig. 15, compound P exhibited thermal stability at least up to about 650 °C wherein two local stability zones spanning temperatures of about 150-310 °C and 380-650 °C could be observed. Further, compound P showed estimated TGA₂₅, TGA₅₀ and TGA₇₅ values of 150 °C, 350 °C and 700 °C, respectively. Thus, compound P would be viewed by those skilled in the art as an excellent candidate for retarding flames under a similar set of conditions.

### Example 8

### Preparation of compounds D-S, E-S1, E-S2, J-S1, J-S2, N-S, O-S and P-S, and their thermal stabilities relative to the corresponding neutral compounds as determined via TGA

Several phosphate, polyphosphate and phosphonate salts of melamine compounds of the invention were prepared and characterized as discussed further below to determine if these would provide a synergistic effect with respect to the thermal stability afforded by the melamine moieties present in the compounds, and hence obviate the need to use additional commercial synergists when manufacturing products comprising compounds of the invention.

As will be appreciated by those of skill in the art, although the following describes the preparation and characterization of select phosphorous-containing salts of the non-halogenated melamine compounds of the invention, these are but a representative sample from a large number of possible salts which, as may be envisioned by a skilled person, could be prepared from the compounds of the invention. Moreover, as a skilled person surely knows, the full effect that a phosphorus counter ion, or any counter ion for that matter, may have on the heat stability of a given melamine compound of the invention cannot be predicted solely based on TGA features (e.g., heat stability zones) of the individual components of the salt (viz., in their neutral form) alone, as indeed, only in some materials was a pronounced change in TGA profile observed. Similarly, while some hypothetical salts may exhibit undesirable changes in their TGA profile compare to the neutral forms, in some instances the profile changed unexpectedly to the point where several more stability zones in the salt appeared to spread over a wider range of temperatures when compared to that of the corresponding neutral melamine compound. As such, the behavior of the TGA curve recorded for melamine salts of the invention cannot be predicted a priori and it necessitates undue experimentation in order to ascertain which salts would afford superior stability and which would not, as discussed in more detail below.

### General Synthetic Procedure

A non-halogenated multi-melamine compound of the invention was suspended in distilled water and heated to 100°C. A solution of the phosphorus-containing acid in distilled water was added dropwise and the mixture was heated at 100 °C and stirred vigorously for 1-3 hours. Upon cooling, the phosphorous-containing salt of the multi-melamine compound was separated by filtration, washed successively with distilled water and dried. Among the acids used were phosphoric acid (see, for example, Scheme 9), polyphosphoric acid and phenylphosphonic acid.

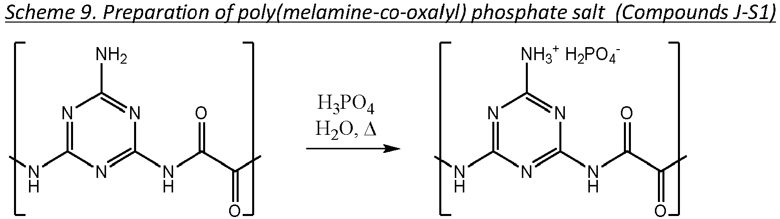

### Characterization

The resulting salts were characterized as follows by attenuated total reflection-Fourier transform infrared spectroscopy (ATR-FTIR), scanning electron microscopy-with energy dispersive X-ray analysis (SEM-EDX) and TGA.

ATR-FTIR spectra of the salts exhibited new strong signals at ^{~}1000 cm⁻¹, indicative of the phosphate groups (data not shown).

SEM-EDX also confirmed the presence of phosphorus in all salt samples (data not shown).

The thermal stabilities of the salts were evaluated via TGA, the results of which are shown in Figs. 17-24 and discussed further below:

As shown in Fig. 17, compared to the TGA of the corresponding neutral compound J shown in Fig. 8, the poly(melamine-co-oxalyl) phosphate salt (compound J-S1) exhibited significant and nearly continuous thermal stability up to about 725 °C where at least 3 more overlapping stability zones could be observed to span this temperature range. Further, compound J-S1 showed estimated TGA₂₅, TGA₅₀ and TGA₇₅ values of 320 °C, 480 °C and 670 °C, respectively, which were elevated relative to those of compound J overall.

As shown in Fig. 23, compared to the TGA of the corresponding neutral compound J shown in Fig. 8, the poly(melamine-co-oxalyl) polyphosphate salt (compound J-S2) also exhibited nearly continuous thermal stability but only up to about 550 °C, which is about 100 degrees less than that observed for compound J. Accordingly, compound J-S2 showed estimated TGA₂₅, TGA₅₀ and TGA₇₅ values of 200 °C, 380 °C and 540 °C, respectively, which were reduced relative to compound J overall. Nevertheless, at least 3 more overlapping stability zones could be observed in compound J-S2 to span this thermally stable temperature range.

As shown in Fig. 18, compared to the TGA of the corresponding neutral compound O shown in Fig. 14, the poly(melamine-co-tolyl urea) phosphate salt (compound O-S) exhibited significant thermal stability up to about 900 °C where three extended stability zones spanning temperatures of about 30-300 °C, 350-500 °C and 550-900 °C could be observed. Further, compound O-S showed estimated TGA₂₅, TGA₅₀ and TGA₇₅ values of 310 °C, 330 °C and 530 °C, respectively, which were elevated relative to those of compound O overall.

As shown in Fig. 19, compared to the TGA of the corresponding neutral compound P shown in Fig. 15, the poly(melamine-co-bisphenylmethylene urea) polyphosphate salt (compound P-S) exhibited extended thermal stability up to about 900 °C where three extended stability zones spanning temperatures of about 100-300 °C, 350-500 °C and 550-900 °C could be observed. Further, compound P-S showed estimated TGA₂₅, TGA₅₀ and TGA₇₅ values of 300 °C, 340 °C and 850 °C, respectively, which were elevated relative to those of compound P overall.

As shown in Fig. 20, compared to the TGA of the corresponding neutral compound D shown in Fig. 10, the 3-melaminepropyl silica polyphosphate salt (compound D-S) exhibited extended thermal stability up to at least about 900 °C where two extended stability zones spanning temperatures of about 100-400 °C, 550-900 °C could be observed. Further, compound D-S showed estimated TGA₂₅, TGA₅₀ and TGA₇₅ values of 340 °C, 510 °C and greater than 900 °C, respectively, which were elevated relative to those of compound D overall.

As shown in Fig. 21, compared to the TGA of the corresponding neutral compound E shown in Fig. 4, the trimelamine benzene tricarboxamide polyphosphate salt (compound E-S1) exhibited robust thermal stability up to at least about 900 °C where at least 3 more overlapping stability zones could be observed to span this temperature range. Further, compound E-S1 showed estimated TGA₂₅, TGA₅₀ and TGA₇₅ values of 330 °C, 490 °C and 620 °C, respectively, which were elevated relative to those of compound E overall.

As shown in Fig. 22, compared to the TGA of the corresponding neutral compound E shown in Fig. 4, the trimelamine benzene tricarboxamide phenylphosphonate salt (compound E-S2) exhibited robust thermal stability up to at least about 900 °C where at least 3 more overlapping stability zones could be observed to span this temperature range. Further, compound E-S2 showed estimated TGA₂₅, TGA₅₀ and TGA₇₅ values of 300 °C, 430 °C and 580 °C, respectively, which were elevated relative to those of compound E overall.

As shown in Fig. 24, compared to the TGA of the corresponding neutral compound N shown in Fig. 13, the poly(melamine-co-isophorone urea) phenylphosphonate salt (compound N-S) exhibited thermal stability up to about 450 °C wherein three local stability zones spanning temperatures of about 30-75 °C, 125-300 °C and 350-425 °C could be observed. Further, compound N-S showed estimated TGA₂₅, TGA₅₀ and TGA₇₅ values of 90 °C, 310 °C and 420 °C, respectively, which were somewhat reduced relative to those of compound N overall.

## Claims

1. Use of non-halogenated melamine compounds as flame retardants, **characterized by** having a TGA₂₅ value of at least 200 degrees Centigrade, a TGA₅₀ value of at least 300 degrees Centigrade, or a TGA₇₅ value of at least 500 degrees Centigrade, as measured on a thermogravimetric analysis (TGA) curve produced at a heating rate of 10 degrees Centigrade per minute and at a 60 mL/min oxygen or nitrogen flow, and wherein said compounds are small molecules, linear or branched polymers, or salts thereof, wherein the compounds are selected from:
| compound | A | Y | X | n |
|---|---|---|---|---|
| 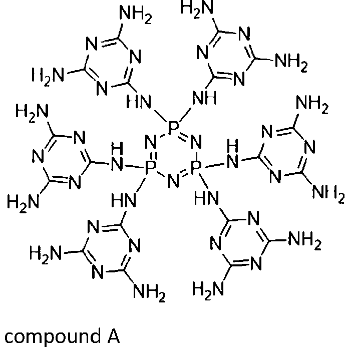 | 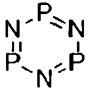 | absent | melamine moiety | 6 |
| 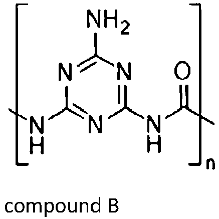 | absent | C=O | melamine moiety | 1-100 |
| 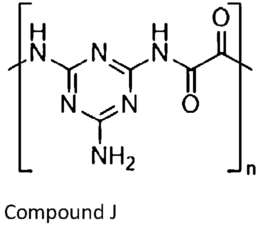 | absent | oxalyl | melamine moiety | 1-100 |
| 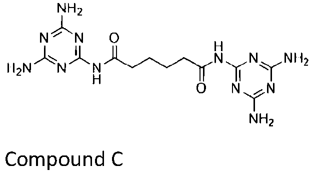 | n-butyl | C=O | melamine moiety | 2 |
| 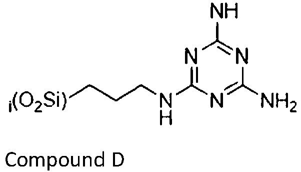 | absent | 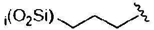 | melamine moiety | 1 |
| 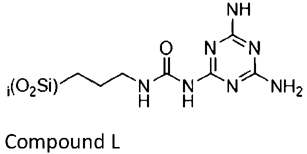 | absent | 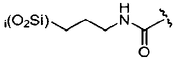 | melamine moiety | 1 |
| 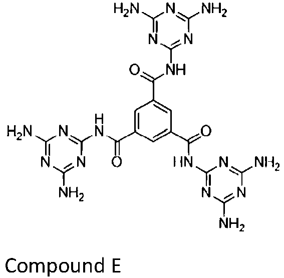 | 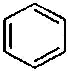 | C=O | melamine moiety | 3 |
| 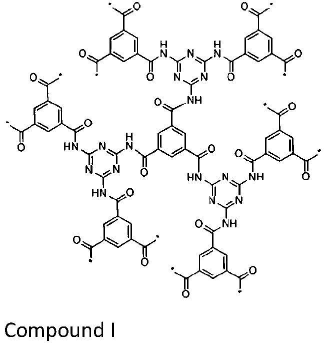 | 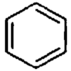 | C=O | X is Xₘ, | 3 |
| wherein the asterisk (*) denotes the point of attachment in the repeating unit to which additional X groups having a structure according to formula (II) are added. | | | | |
| wherein Xₘ is a branched repeating unit of melamine having a structure according to formula (II) , m is an integer from 1-500. | | | | |
| 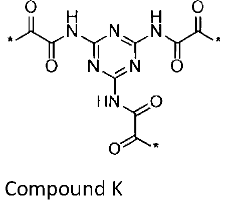 wherein the asterisk (*) denotes the point of attachment in the repeating unit to which additional X groups having a structure according to formula (III) are added. | absent | absent | X = Xₘ | |
| wherein Xₘ is a branched repeating unit of a melamine moiety having a structure according to formula (III): | | | | |
| 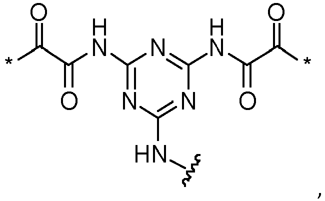 and m is an integer from 1-500 | | | | |
| 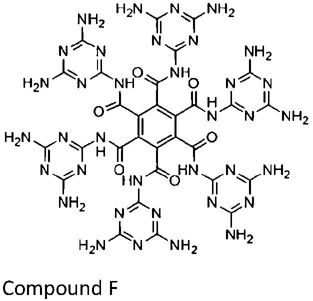 | 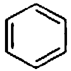 | C=O | melamine moiety | 6 |
or wherein the compound is a salt comprising a positively charged melamine cation and a negatively charged phosphorous-containing anion, the salt compounds are selected from:
(a) a phosphate salt of compound J: 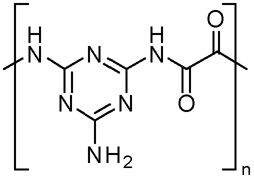 having the structure below: 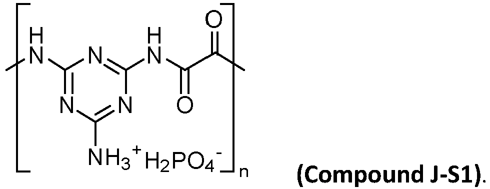
(b) a polyphosphate salt of compound J: 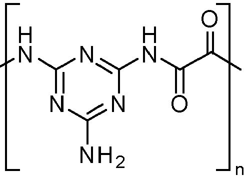 having the structure below: 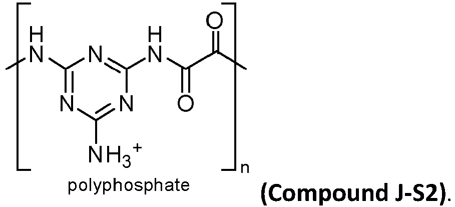
(c) a polyphosphate salt of compound D: 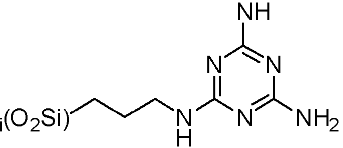 having the structure below: 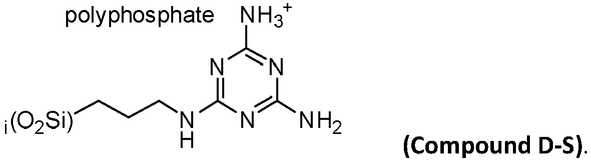
(d) a polyphosphate salt of compound E: 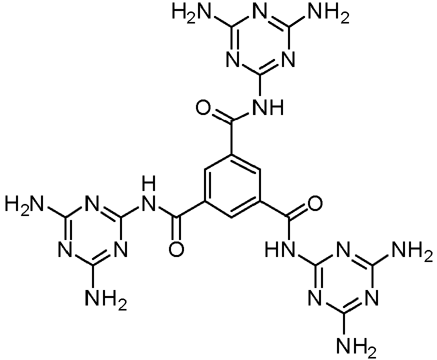 having the structure below: 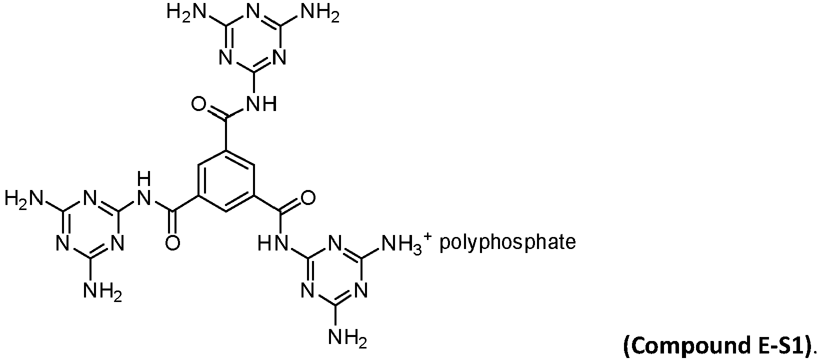
(e) a phenylphosphonate salt of compound E: 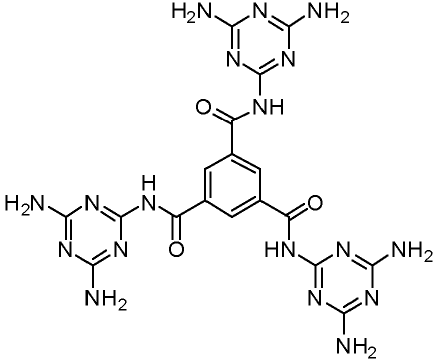 having the structure below:

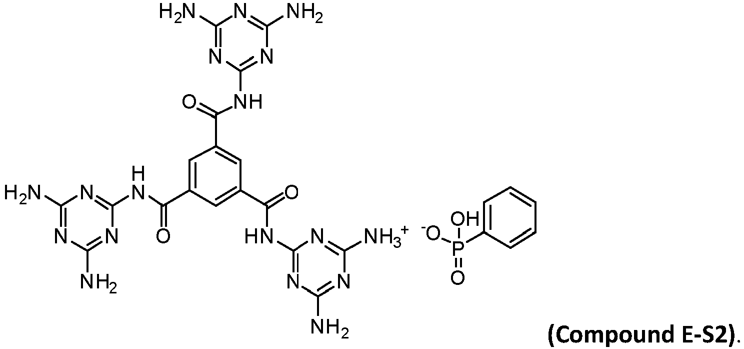

2. A flame retarded plastic material comprising one or more of the compounds according to claim 1, optionally in combination with other flame retardants.

3. An article of manufacture made of or coated with one or more of the compounds according to claim 1, optionally in combination with other flame retardants.

4. The flame retarded plastic material according to claim 2 or an article according to claim 3, further comprising additives such as synergists, stabilizers, such as tridecylphosphite, barium-cadmium soaps and organo tin compounds, inorganic fillers, plasticizers, such as terephthalates, antioxidants, lubricants or colorants.

5. Compositions comprising one or more non-halogenated melamine compounds according to claim 1, optionally in combination with other flame retardants, wherein about 0.1 - 50 percent by weight of the composition consists of said melamine compounds.

6. The composition of claim 5, wherein about 5 - 30 percent by weight of the composition consists of said melamine compounds.

## Patentansprüche

1. Verwendung von nicht-halogenierten Melaminverbindungen als Flammschutzmittel, **dadurch gekennzeichnet, dass** sie einen TGA₂₅-Wert von mindestens 200 Grad Celsius, einen TGA₅₀-Wert von mindestens 300 Grad Celsius oder einen TGA₇₅-Wert von mindestens 500 Grad Celsius aufweisen, gemessen an einer Kurve der thermogravimetrischen Analyse (TGA), die bei einer Heizrate von 10 Grad Celsius pro Minute und bei einem Sauerstoff- oder Stickstoffstrom von 60 mL/min erstellt wurde, und wobei die Verbindungen kleine Moleküle, lineare oder verzweigte Polymere oder Salze davon sind, wobei die Verbindungen ausgewählt sind aus:
| Verbindung | A | Y | X | n |
|---|---|---|---|---|
| 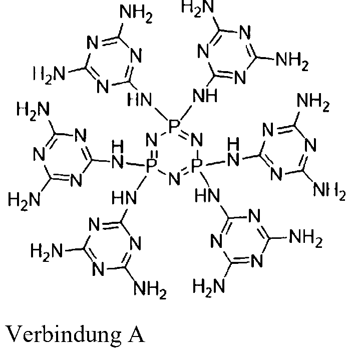 | 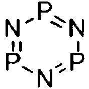 | abwesend | Melamineinheit | 6 |
| 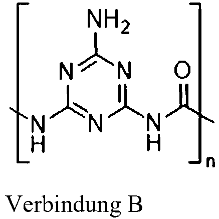 | abwesend | C=O | Melamineinheit | 1-100 |
| 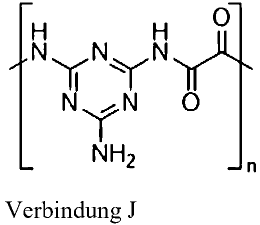 | abwesend | Oxalyl | Melamineinheit | 1-100 |
| 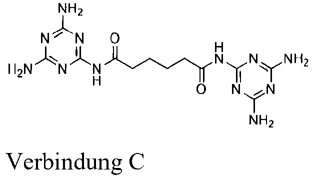 | n-Butyl | C=O | Melamineinheit | 2 |
| 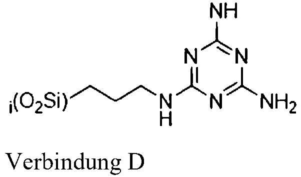 | abwesend | 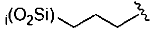 | Melamineinheit | 1 |
| 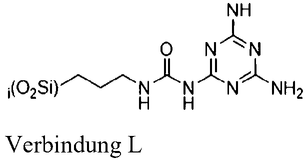 | abwesend | 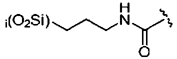 | Melamineinheit | 1 |
| 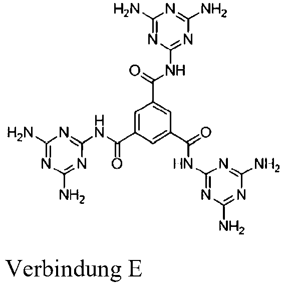 | 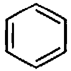 | C=O | Melamineinheit | 3 |
| 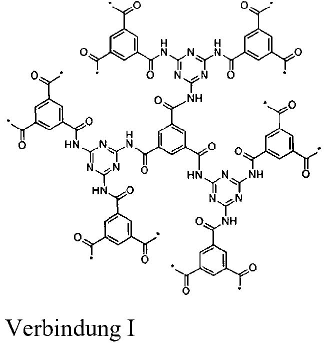 | 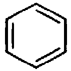 | C=O | X ist Xₘ, | 3 |
| wobei das Sternchen (*) die Anknüpfungsstelle in der Wiederholungseinheit bezeichnet, an der zusätzliche X-Gruppen mit einer Struktur gemäB Formel (II) angefügt werden. | | | | |
| wobei Xₘ eine verzweigte Wiederholungseinheit von Melamin mit einer Struktur gemäß Formel (II) ist und m eine ganze Zahl von 1-500 ist. | | | | |
| 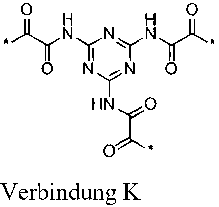 wobei das Sternchen (*) die Anknüpfungsstelle in der Wiederholungseinheit bezeichnet, an der zusätzliche X-Gruppen mit einer Struktur gemäß Formel (III) angefügt werden. | abwesend | abwesend | X = Xₘ | |
| wobei Xₘ eine verzweigte Wiederholungseinheit einer Melamineinheit mit einer Struktur gemäß Formel (III): | | | | |
| 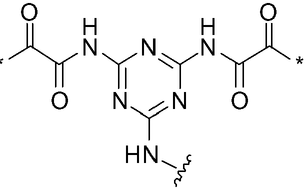 ist und m eine ganze Zahl von 1-500 ist | | | | |
| 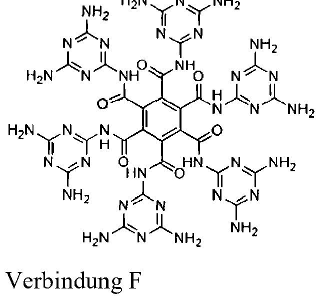 | 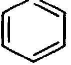 | C=O | Melamineinheit | 6 |
oder wobei die Verbindung ein Salz ist, umfassend ein positiv geladenes Melaminkation und ein negativ geladenes phosphorhaltiges Anion, wobei die Salzverbindungen ausgewählt sind aus:
(a) einem Phosphatsalz der Verbindung J: 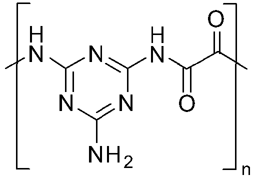 mit der nachstehenden Struktur: 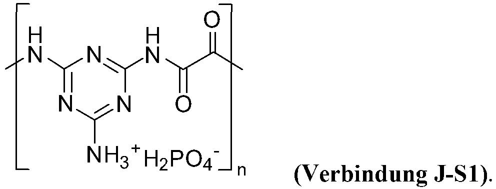
(b) einem Polyphosphatsalz der Verbindung J: 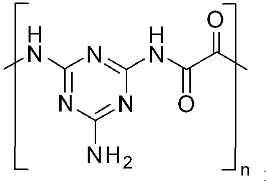 mit der nachstehenden Struktur: 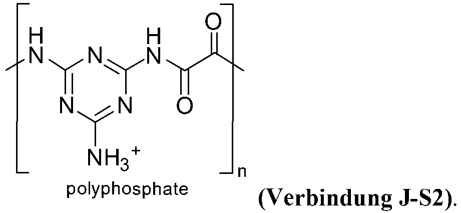
(c) einem Polyphosphatsalz der Verbindung D: 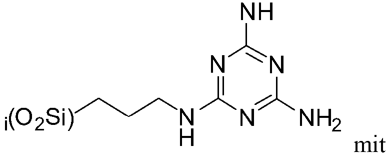 der nachstehenden Struktur: 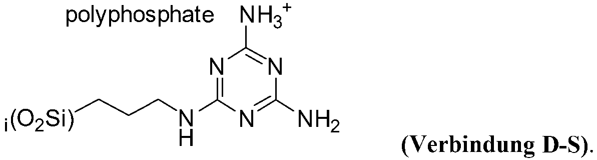
(d) einem Polyphosphatsalz der Verbindung E: 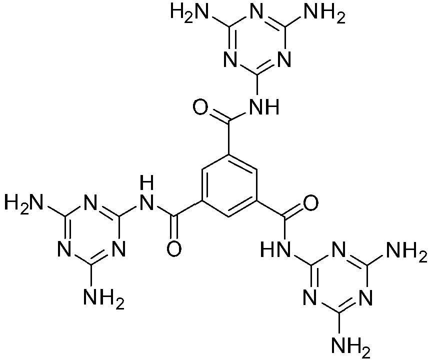 mit der nachstehenden Struktur: 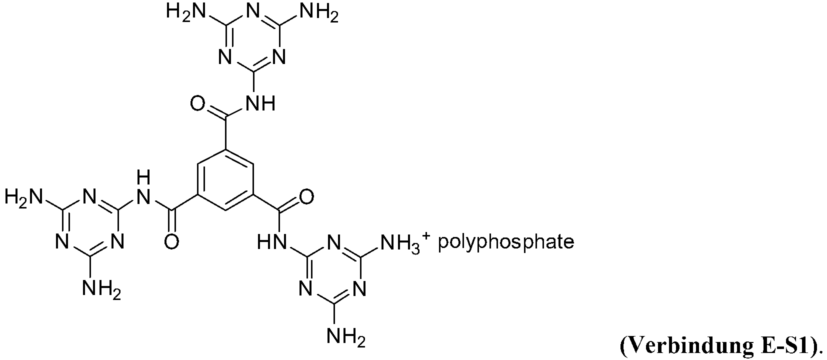
(e) einem Phenylphosphonatsalz der Verbindung E: 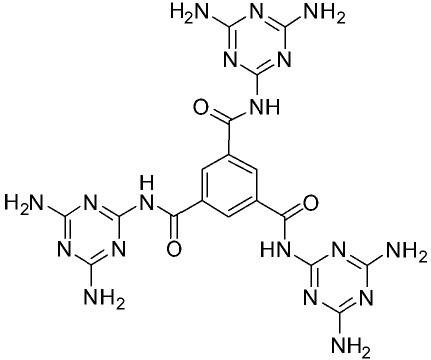 mit der nachstehenden Struktur:

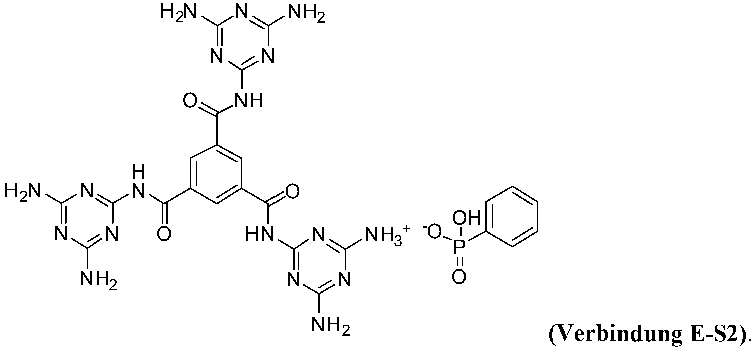

2. Flammgeschütztes Kunststoffmaterial, umfassend eine oder mehrere der Verbindungen nach Anspruch 1, gegebenenfalls in Kombination mit anderen Flammschutzmitteln.

3. Herstellungsgegenstand, hergestellt aus oder beschichtet mit einer oder mehreren der Verbindungen nach Anspruch 1, gegebenenfalls in Kombination mit anderen Flammschutzmitteln.

4. Flammgeschütztes Kunststoffmaterial nach Anspruch 2 oder Gegenstand nach Anspruch 3, ferner umfassend Additive wie Synergisten, Stabilisatoren, wie Tridecylphosphit, Barium-Cadmium-Seifen und Organozinnverbindungen, anorganische Füllstoffe, Weichmacher, wie Terephthalate, Antioxidantien, Gleitmittel oder Farbmittel.

5. Zusammensetzungen, umfassend eine oder mehrere nicht-halogenierte Melaminverbindungen nach Anspruch 1, gegebenenfalls in Kombination mit anderen Flammschutzmitteln, wobei etwa 0,1-50 Gewichtsprozent der Zusammensetzung aus den Melaminverbindungen bestehen.

6. Zusammensetzung nach Anspruch 5, wobei etwa 5-30 Gewichtsprozent der Zusammensetzung aus den Melaminverbindungen bestehen.

## Revendications

1. Utilisation de composés de mélamine non halogénés en tant que retardateurs de flamme, **caractérisés en ce qu'**ils présentent une valeur de TGA₂₅ d'au moins 200 degrés centigrades, une valeur de TGA₅₀ d'au moins 300 degrés centigrades, ou une valeur de TGA₇₅ d'au moins 500 degrés centigrades, telles que mesurées sur une courbe d'analyse thermogravimétrique (TGA) produite à une vitesse de chauffage de 10 degrés centigrades par minute et sous un débit d'oxygène ou d'azote de 60 mL/min, et dans laquelle lesdits composés sont de petites molécules, des polymères linéaires ou ramifiés, ou des sels de ceux-ci, dans laquelle les composés sont choisis parmi :
| composé | A | Y | X | n |
|---|---|---|---|---|
| 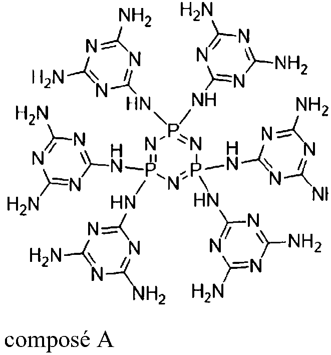 | 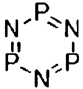 | absent | fragment mélamine | 6 |
| 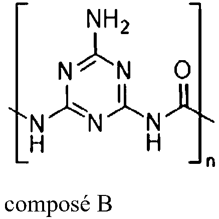 | absent | C=O | fragment mélamine | 1-100 |
| 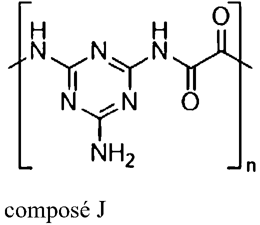 | absent | oxalyle | fragment mélamine | 1-100 |
| 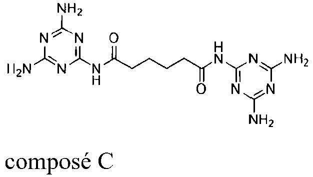 | n-butyle | C=O | fragment mélamine | 2 |
| 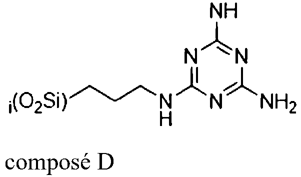 | absent | 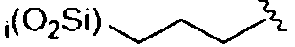 | fragment mélamine | 1 |
| 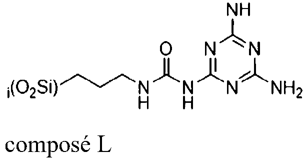 | absent | 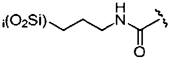 | fragment mélamine | 1 |
| 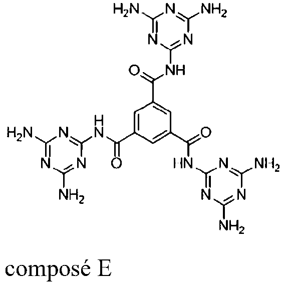 | 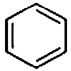 | C=O | fragment mélamine | 3 |
| 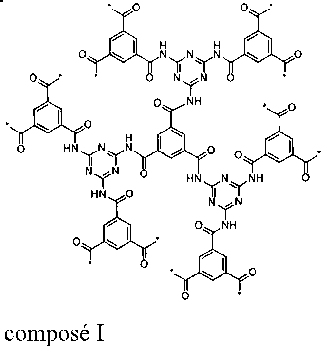 | 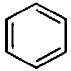 | C=O | X est Xₘ, | 3 |
| dans lequel l'astérisque (*) désigne le point de fixation dans l'unité répétitive à laquelle des groupes X supplémentaires ayant une structure selon la formule (II) sont ajoutés. | | | | |
| dans lequel Xₘ est une unité répétitive ramifiée de mélamine ayant une structure selon la formule (II) , m est un nombre entier de 1 à 500. | | | | |
| 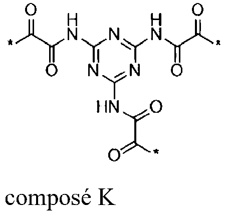 dans lequel l'astérisque (*) désigne le point de fixation dans l'unité répétitive à laquelle des groupes X supplémentaires ayant une structure selon la formule (III) sont ajoutés. | absent | absent | X = Xₘ | |
| dans lequel Xₘ est une unité répétitive ramifiée d'un fragment mélamine ayant une structure selon la formule (III) : * 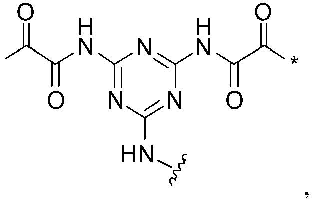 et m est un nombre entier de 1 à 500 | | | | |
| 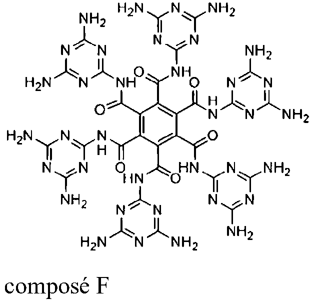 | 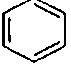 | C=O | fragment mélamine | 6 |
ou dans laquelle le composé est un sel comprenant un cation mélamine chargé positivement et un anion contenant du phosphore chargé négativement, les composés de type sel étant choisis parmi :
(a) un sel de phosphate du composé J : 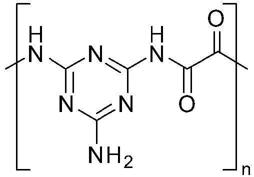 ayant la structure ci-dessous : 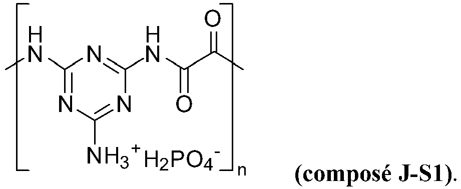
(b) un sel de polyphosphate du composé J : 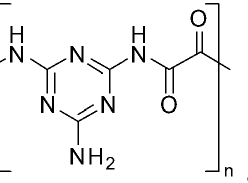 ayant la structure ci-dessous : 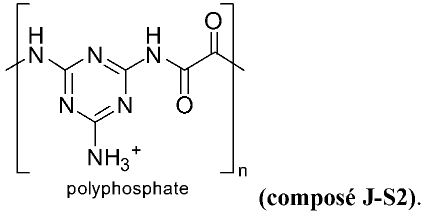
(c) un sel de polyphosphate du composé D : 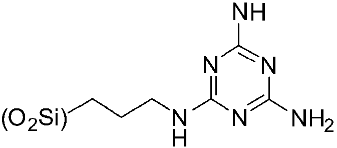 ayant la structure ci-dessous : 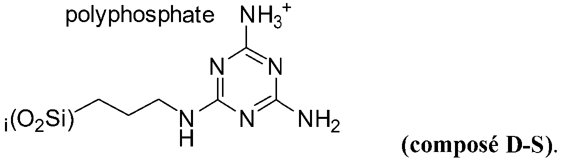
(d) un sel de polyphosphate du composé E : 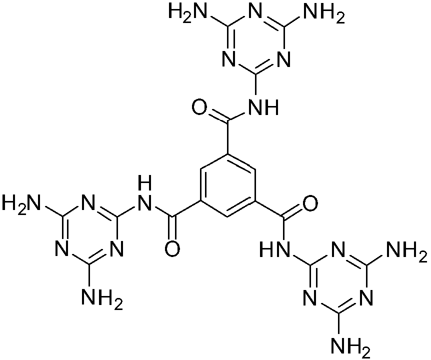 ayant la structure ci-dessous : 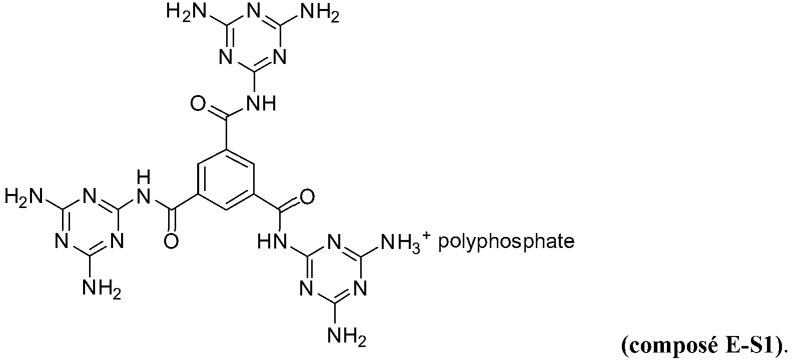
(e) un sel de phénylphosphonate du composé E : 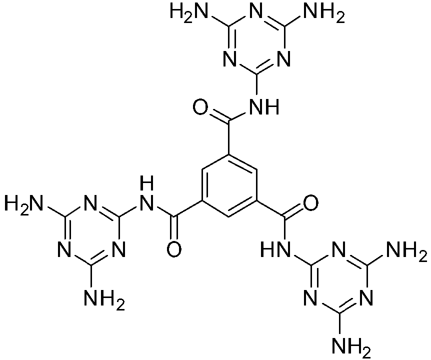 ayant la structure ci-dessous :

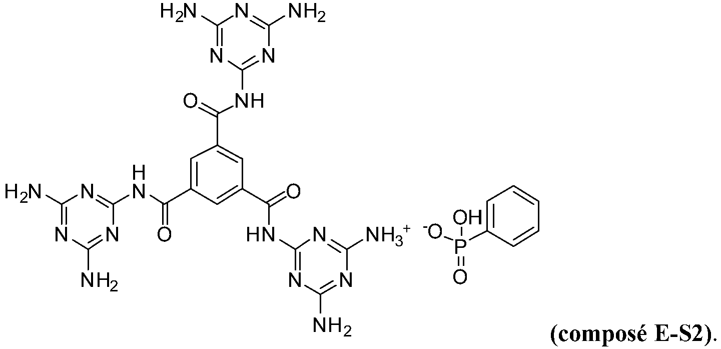

2. Matériau plastique ignifugé comprenant un ou plusieurs des composés selon la revendication 1, éventuellement en combinaison avec d'autres retardateurs de flamme.

3. Article manufacturé réalisé à partir d'un ou plusieurs des composés selon la revendication 1, ou revêtu de ceux-ci, éventuellement en combinaison avec d'autres retardateurs de flamme.

4. Matériau plastique ignifugé selon la revendication 2 ou article selon la revendication 3, comprenant en outre des additifs tels que des synergistes, des stabilisants, tels que le phosphite de tridécyle, les savons de baryum-cadmium et les composés organostanniques, des charges inorganiques, des plastifiants, tels que les téréphtalates, des antioxydants, des lubrifiants ou des colorants.

5. Compositions comprenant un ou plusieurs composés de mélamine non halogénés selon la revendication 1, éventuellement en combinaison avec d'autres retardateurs de flamme, dans lesquelles environ 0,1 à 50 pour cent en poids de la composition sont constitués desdits composés de mélamine.

6. Composition selon la revendication 5, dans laquelle environ 5 à 30 pour cent en poids de la composition sont constitués desdits composés de mélamine.
